(19)

Europäisches
Patentamt
European
Patent Office
Office européen
des brevets

(11) **EP 3 918 985 A1**

(12) **EUROPEAN PATENT APPLICATION**

(43) Date of publication:
**08.12.2021 Bulletin 2021/49**

(51) Int Cl.:
**A61B 5/0476** (2006.01)          **A61B 5/00** (2006.01)

(21) Application number: **20178463.4**

(22) Date of filing: **05.06.2020**

(84) Designated Contracting States:
**AL AT BE BG CH CY CZ DE DK EE ES FI FR GB GR HR HU IE IS IT LI LT LU LV MC MK MT NL NO PL PT RO RS SE SI SK SM TR**
Designated Extension States:
**BA ME**
Designated Validation States:
**KH MA MD TN**

(71) Applicant: **Oncomfort SA**
**1300 Wavre (BE)**

(72) Inventors:
• **Jooris, Diane**
**3080 Tervuren (BE)**
• **Huyghe, Mario**
**8792 Desselgem (BE)**
• **Toussaint, Clémence**
**4210 Oteppe (BE)**

(74) Representative: **De Clercq & Partners**
**Edgard Gevaertdreef 10a**
**9830 Sint-Martens-Latem (BE)**

(54) **A METHOD AND SYSTEM FOR MONITORING A LEVEL OF PAIN**

(57)     There is described a computer-implemented method for measurement of a level of pain. The method comprises the steps of:

- receiving measured data comprising electroencephalogram, EEG, data collected from one or more EEG electrodes;

- extracting from the EEG data, a group 4 indicator corresponding to:

- a power, power (ta), associated with a theta-alpha frequency band, ta, within a theta-alpha frequency range; and

- determining, based on said group 4 indicator, a level of pain, LoP, which is a value indicative for the level of pain in the subject.

EP 3 918 985 A1

**Description**

Field of the invention

The present invention is in a field of a computer-implemented method for automatically monitoring a level of pain as perceived by a subject.

Background to the invention

Pain and the impact of the related suffering on the quality of life is a well-known and universal problem. Pain management is thus an important branch of medicine. However prior art methods for assessment of the level of pain a subject is experiencing have important drawbacks and are not able to provide for an efficient, reliable, robust, objective and reproducible indication of the level of pain of a subject. This problem is immediately apparent when the subject is not capable of providing for a reliable or clear description of the level of pain experienced, such as for example in the case of non-verbal children, persons with a disability, ....

Accurately measuring pain is important in a medical setting. The pain measurement is for example used as a screening tool, for example to screen for potential diseases or medical problems, or to perform a triage by determining the urgency of the needs of one patient over another. Pain measurements help to determine an accurate diagnosis, a treatment plan, and evaluate the effectiveness of treatment.

Additionally, there is a growing problem of pain treatment overdosing, such as for example prescription pain killer overdosing, and care workers are in need of more reliable tools in order to provide for a safe and effective pain treatment. A robust and reliable measurement of the level of pain of a subject could thus provide for a safer and more effective pain treatment, thereby reducing the risk for overdosing and/or underdosing, and thus increasing the quality of life of the subject in a safe way.

A patient's self-reported pain is currently a critical tool in pain measurement, as it is the only tool that is able to provide for a measure of the level of pain as perceived by the patient, which is generally known as the most valid measure of pain. Currently in order to assess this level of pain as perceived by the patient, use is made of pain scales that assist in manually determining the level of pain as perceived by the patient by means of self-reporting. However it is clear that such manual tools that rely on self-reported assessment of the patient are prone to undesired subjective bias and lack efficiency, reliability, precision and reproducibility.

As for example detailed in "Assessment of Patient Comfort During Palliative Sedation: Is it always Reliable?" (Deschepper, Bilsen, Laureys, 2014), prior art pain measurement systems have limitations. Current situation and challenges Awareness/ non awareness of conscious perception of pain is sometimes mistakenly considered. The level of consciousness of a patient can be modified pharmacologically (e.g. pharmacological), due a pathology (e.g. brain injury...) or psychophysiologically (e.g. clinical hypnosis). For patients with such a reduced level of consciousness, there is no evidence that current assessments of awareness and suffering are accurate. This holds especially for non-verbal patients or patients with in a coma or sedated situation. Titration of drugs is further also a challenge. During a surgery, analgesic and/or sedation dosages are given by default (xxx mg/kg) and titrated based on estimated general awareness levels (behavioural assessment/ BIS monitoring/ ...). However, there is no way of knowing what level of conscious perception of pain is present for the subject. This leads to risks of overdosing and/or underdosing of for example analgesics and/or sedatives. It is further difficult to objectively and/or robustly verify the principle of proportionality in cases such as for example palliative sedation, for example for non-verbal patients or for patients in a modified state of consciousness, due to for example drugs or psychological intervention. A further overview of known analgesia nociception measurement systems is for example also described in the Oxford Textbook of Anaesthesia (Jonathan G. Hardman, Philip M. Hopkins, Michel M. R. F. Struys, Oxford University Press, 2017, ISBN: 0199642044, 9780199642045) in table 26.1 on page 451. Similarly as discussed above, BIS is a method measuring general awareness. It is particularly noted in the section "EEG arousal response" on that page that an index calculated from EEG data, as example the BIS Index, and EMG power has only moderate value in predicting responses to noxious stimulation. In addition, also other parameters that provide indicators for monitoring nociception, such as for example pupillary dilation, sweat secretion, etc. , do not provide for a reliable indication of the level of pain as consciously perceived by the subject. In this way, it is clear that know nociception indexes which quantify a patient's physiological pain response or nociception are not able to provide a reliable indication of the level of pain as consciously perceived by the subject. These known nociception indexes only make use of signals derived from physiological pain responses, such as pupillary dilation, saliva flow, breathing rate, blood pressure, heart rate, etc. which are nociception-related physiological responses resulting from activation of the sympathetic nervous system. Such involuntary and/or unconsiouss physiological responses, however do not provide for a reliable indication of the level of pain as consciously perceived by the subject. Such physiological responses may also be triggered in response to other events then nociception, thereby limiting their reliability as robust indicators for the level of pain. One known example is the known NOL index or Nociception Level index, which is an index developed by the company Medasense to quantify a patient's physiological pain response or nociception based on one or more of the following physiological signals: breathing rate, blood pressure, heart rate, muscle tension, skin temperature and sweating. Another example is for example ANI or Analgesia nociception index, which is based on electrocardiographic data reflecting parasympathetic activity, which as explained above only relates to an involuntary and/or unconscious physiological response to nociception, and could be triggered in response to other events then nociception and is thus not able to provide a reliable indication of the level of pain as consciously perceived by the subject.

The management of pain in general, and specifically in clinical or therapeutic procedures is usually done through pharmacological solutions. Pharmacological analgesic treatment and/or pharmacological sedation presents risks, which are incremental in presence of several factors such as age, respiratory or cardiac pathologies, obesity. Excessive sedation used (conscious/ unconscious sedation, general anesthesia) through drugs increase the risk/ incidence of moderate to severe adverse events. Monitoring systems (such as Bis and Entropy) are designed for pharmacological sedation and correlate poorly with clinical parameters of depth of dominantly non-pharmacologically induced sedation/anesthesia. Non-pharmacological sedation has been practiced for decades (hypnosedation / clinical hypnosis / hypnotherapy) using hypnosis delivered one-on-one or via playback of recorded session, optionally with locoregional anesthesia (LRA) if needed for local/ regional pain management and/or conscious IV sedation. Changes in brain activity in hypnotic state have been objectivized by brain imagery (fMRI and/ or EEG), but so far no objective quantification has been available to measure, objectivate and, based on these, manage patient's level of pain as consciously perceived by the patient, for example during a hypnotic state or any other treatment involving a modified state of consciousness, such as for example pharmacological sedation. There is no existing way to quantify or predict patient's physiological/ psychological/ behavioural reaction to painful stimuli and modulation of conscious perception of nociceptive messages in a modified state of consciousness, such as for example in the case of pharmacological or non-pharmacological sedation. There are currently no reliable tools to objectively monitor/trend/assess/predict a patient's level of pain as consciously perceived.

Current nociception measurements do not measure the conscious perception of pain and can lead to overdosing, such as for example overdosing of analgesic drugs, overdosing of sedatives, .... Nociception can trigger responses without necessarily causing the conscious perception of pain. Nociception measurements could also lead to underestimation of level of pain since pain can occur in the absence of nociceptive input, e.g. due to a dysfunction of autonomous nervous system.

There exists in the art a need for an improved, robust and reliable measurement of the level of pain as consciously perceived by the subject, preferably allowing to arrive at a standard scale or index, useful for an assessment of the subject by a medical practitioner or caregiver. Additionally, there is specifically also a need for an improved, robust and reliable measurement of the level of pain as consciously perceived by the subject when the subject for example experiences a modified state of consciousness,

## Summary

According to a first aspect of the invention, there is provided a computer-implemented method for measurement of a level of pain, the method comprising the steps of:

- receiving measured data comprising electroencephalogram, EEG, data, for example collected from one or more EEG electrodes;
- extracting from the EEG data, a group 4 indicator corresponding to:

  - a power, power (ta), associated with a theta-alpha frequency band, ta, within a theta-alpha frequency range; and

- determining, based on said group 4 indicator, a level of pain, LoP, which is a value indicative for the level of pain in the subject.

This allows for an automated, efficient, accurate, reproducible, and objectivized determination and/or monitoring of the level of pain as consciously perceived by the subject. This improved pain measurement then helps in determining a more accurate diagnosis, an improved treatment plan, and an improved evaluation the effectiveness of treatment. This robust and reliable measurement of the level of pain of as perceived by the subject also provides for a safer and more effective pain treatment, thereby reducing the risk for overdosing and/or underdosing, and thus increasing the quality of life of the subject in a safe way. In this way the use of pharmacological sedatives, analgesics, ... can be optimized.

It is clear that the level of pain relates to the perceived level of pain, or in other words the consciously perceived level of pain by a subject as measured at the level of the cortex of the brain of the subject.

This for example allows to dose local and/or IV medication to improve and optimize a pain or sedative treatment. If a patient is doing fine during a treatment, and at one point there is measured an increase or a peak in the level of pain, a local anesthetic, and/or IV analgesics dosages could be increased based on objectivized level of pain as consciously perceived by the subject and not on a subjective estimation of patient's pain. Measurement of the LoP also allows for a pain diagnostic for non-verbal patients (children, adults who are disabled, patients with dementia, ...). The measured LoP is for example also a good indicator of the need or not for more sedation: if patient is in an adequate altered state of consciousness, pain is not consciously perceived and an increased level of sedation is not required.

Such an improved measurement of the level of pain is advantageous in the following specific clinical cases:

- A patient is undergoing a procedure with light sedation. Based on the measured LoP, the Anesthesiologist will be able to objectively monitor level of pain to adapt sedation if needed. As example, increasing IV sedation dosages could be replaced by increasing local anesthesia dosage as anesthesiologist will be able to monitor if local anesthesia is enough to manager patient's pain perception
- Non-verbal patients (palliative, injured...) will be able to undergo a procedure with an objective monitoring of the level of pain, as consciously perceived by the patient, related to the procedure. Better pain management will be possible.
- Limitation of sedation or analgesia will be possible if the patient does not need it. The observational scales used during procedures (such as Behavioral Pain Scale or BPS) are not reliable: a patient could be gowning or frowning with no conscious perception of pain. The measured LoP allows to limit titration of sedation when extra sedation is not objectively needed.
- Objective Measure of efficacy of non-pharmacological pain management treatments (such as clinical hypnosis, digital sedation, acupuncture...) during painful moments of a treatment.

According to an embodiment, there is provided a method, wherein the theta-alpha frequency band, ta, comprises one or more of the following:

- a frequency band encompassing both theta and alpha brain waves;
- a frequency band extending from 6Hz up to and including 12Hz; and/or
- a bandwidth of at least 2Hz and a frequency band comprising at least fast theta waves extending from 6Hz up to and including 8Hz, and/or

wherein the theta-alpha frequency range:

- comprises a frequency range encompassing both theta and alpha brain waves; and/or
- extends from 4 Hz up to and including 12 Hz.

According to an embodiment, there is provided a method, comprising the step of:

- extracting from the EEG data, a group 3 indicator corresponding to:

    - a mean signal peak-to-peak amplitude, MSPA; and

- determining the LoP based on the group 4 indicator and at least the group 3 indicator.

According to an embodiment, there is provided a method, comprising the step of:

- receiving measured data comprising electromyography, EMG, data, for example collected from one or more EMG electrodes or any other suitable electrode;
- extracting from the EMG data, a group 5 indicator corresponding to:

    - a mean signal peak-to-peak amplitude, EMG-MSPA; and

- determining the LoP based on the group 4 indicator and at least the group 5 indicator.

According to an embodiment, there is provided a method, comprising the steps of:

- determining the LoP based on the group 4 indicator, the group 3 indicator, and the group 5 indicator.

According to an embodiment, there is provided a method, wherein the method comprises the step of:

- receiving the measured data of the subject comprising the electroencephalogram, EEG, data collected from one or more of the following EEG electrodes:

  - a frontal (F) EEG electrode configured for collection of F-EEG electrode data from the scalp anatomical region corresponding to a frontal lobe of the subject,
  - a parietal (P) EEG electrode configured for collection of P-EEG electrode data from the scalp anatomical region corresponding to a parietal lobe of the subject,
  - a central (C) EEG electrode configured for collection of C-EEG electrode data from the scalp anatomical region corresponding to a precentral and postcentral gyrus of the subject.

It is clear that further alternative embodiments are possible in which in addition to the EEG electrodes, there is also present a ground electrode and/or a reference electrode. Preferably the data from the EEG electrodes can be processed independently, which means that there is no need to provide for calculations on the interaction and/or interrelation of the signals of two or more EEG electrodes.

According to an embodiment, there is provided a method, wherein the method comprises the steps of:

- receiving reference data comprising measured data of a subject and/or a population correlated to at least one predetermined reference LoP;
- determining at least one correlation of at least one predetermined reference LoP and at least one group indicator extracted from the reference data; and
- scaling and/or indexing a subsequently measured LoP with respect to the at least one predetermined reference LoP by means of said at least one correlation.

A scaled and/or indexed LoP may for example be referred to as a level of pain index or LoPI.

According to a second aspect of the invention, there is provided a computer-implemented method for determining and/or monitoring a level of pain of a subject, comprising the method for measurement of a level of pain according to the first aspect of the invention applied on a subject by receiving the measured data of a subject.

The measured data of the subject could for example be real-time data as being received from suitable sensors, however, alternative embodiments are possible in which historic data, previously stored data, ... and/or any combination thereof is used for measurement of a level of pain.

According to a third aspect of the invention, there is provided computer-implemented method for determining and/or monitoring and/or predicting and/or aggregating a level of pain in a subject before, during and/or after a treatment, wherein the method comprises the steps of:

- measurement of the LoP according to the first aspect of the invention, at at least two different points in time, preferably before, during and/or after the treatment;
- determining and/or monitoring and/or predicting an evolution, preferably a treatment-induced evolution of the LoP, based on a comparison of at least two LoP measured at at least two different points in time;
- aggregating a level of pain score or LoPS based on an aggregation of at least two LoP determined at at least two different points in time.

According to an embodiment, there is provided a method, wherein the method comprises the step of:

- determining the treatment-induced evolution of the LoP, based on a comparison of the LoP measured during and/or after the treatment with the LoP measured before the treatment.

It is clear that according to particular embodiments, the treatment could for example comprise a pain treatment, such as for example a pain-reducing treatment. However it is clear that alternative embodiments of treatments are possible, such as for example a surgical treatment or any other medical intervention during which one or more pain inducing acts are performed, such as for example a needle puncture, placement of a port of a catheter, an incision, introduction of a wire, etc.. During some of these treatments the desired treatment-induced evolution will comprise a reduction of the LoP, however in alternative treatments, it might be more important to monitor the LoP for any undesired changes during the treatment, such that for example the need for extra local anesthesia or other pain management measures could be reliably assessed. However it is clear that such embodiments could be beneficial for pain measurement of a subject under any non-pharmacological and/or pharmacological treatment, such as for example pain treatments, treatments leading to a modified state of consciousness, any other suitable treatment, ....

According to an embodiment, there is provided a method, wherein the treatment is a pain reducing treatment and the method comprises the step of:

- determining and/or monitoring the efficacy of a pain treatment based on the treatment-induced evolution of the LoP.

The pain treatment could for example be a pain-reducing treatment, or alternatively a pain-controlling or pain-preventing treatment that controls or prevents any undesired levels of pain, or any other suitable pain treatment.

According to an embodiment, there is provided a method, wherein the treatment is a pain reducing treatment and the method comprises the step of:

- optimizing and/or adapting a pain treatment based on the treatment-induced evolution of the LoP.

As already mentioned above the pain treatment could for example be a pain-reducing treatment, or alternatively a pain-controlling or pain-preventing treatment that controls or prevents any undesired levels of pain, or any other suitable pain treatment.

According to a fourth aspect of the invention, there is provided a computer-implemented method for determining a treatment and/or determining an adjustment to a treatment and/or determining an intensity of a treatment and/or determining the efficacy of a treatment and/or determining the choice of a treatment, wherein the method comprises the steps of:

- measuring a LoP according to the first aspect of the invention;
- determining a treatment and/or determining an adjustment to a treatment and/or determining an intensity of a treatment based on the measured LoP.

According to a fifth aspect of the invention, there is provided a system configured to measure a level of pain according to the method according to any of the previous aspects of the invention, the system comprising:

- a monitoring apparatus configured to obtain measured data comprising electroencephalogram, EEG, data, for example collected from one or more EEG electrodes;
- a controller module configured for:

  - receiving the measured data from the monitoring apparatus;
  - extracting from the EEG data, a group 4 indicator corresponding to:

    - a power, power (ta), associated with a theta-alpha frequency band, ta, within a theta-alpha frequency range; and

  - determining, based on said group 4 indicator, a level of pain, LoP, which is a value indicative for the level of pain in the subject.

According to an embodiment, there is provided a system, wherein the controller module is further configured for:

- extracting from the EEG data, a group 3 indicator corresponding to:

  - a mean signal peak-to-peak amplitude, MSPA; and

- determining the LoP based on the group 4 indicator and at least the group 3 indicator.

According to an embodiment, there is provided a system, wherein the monitoring apparatus comprises, for obtaining measured data, one or more of:

- one or more EEG electrodes configured for collection of electroencephalogram, EEG, data;
- one or more frontal (F) EEG electrodes configured for collection of F-EEG electrode data from the scalp anatomical region corresponding to a frontal lobe of the subject,
- one or more parietal (P) EEG electrodes configured for collection of P-EEG electrode data from the scalp anatomical region corresponding to a parietal lobe of the subject,
- one or more central (C) EEG electrodes configured for collection of C-EEG electrode data from the scalp anatomical region corresponding to a precentral and postcentral gyrus of the subject;
- optionally an EMG capturing unit;
- optionally a heart rate capturing unit;
- optionally a respiratory data capturing unit.

It is clear that still further alternative embodiments are possible comprising for example one or more sensors and/or capturing units for one or more other electrophysiological and/or physiological measurements.

According to an embodiment, there is provided a system, further comprising a graphical user interface, GUI, configured to indicate numerically and/or graphically one or more of:

- at least two LoP respectively measured at different points in time, preferably a historic, current and/or expected LoP or LoPI
- the evolution of and/or ratio between and/or aggregation of at least two LoP or LoPI measured at at least two different points in time;
- optionally, one or more components of the measured data, preferably one or more EEG data and optionally EMG data.

It is clear that alternative embodiments of the GUI are possible in which further data is displayed, such as for example ECG data, heart rate data, respiratory data, ...

Figure Legends

FIG. 1 shows an embodiment of an experiment for measuring the level of pain as consciously perceived by the subject.

FIG. 2 is a graph indicating a correlation between self-reported pain and computed 6-12 Hz power at EEG electrodes F, P and C.

FIG. 3 shows an embodiment of a computer-implemented method for measuring the level of pain.

FIG. 4 shows an embodiment of a computer-implemented method for determining and/or monitoring a level of pain in a subject before, during and/or after a treatment.

FIG. 5 is a graph indicating the correlation between MSPA measured at the 3 EEG electrodes F, C and P and pain.

FIG. 6 is a graph indicating a correlation between self-reported pain and EMG-ERP amplitude.

FIGs. 7 to 14 illustrate examples of outputs to a graphical user interface.

FIG. 15 illustrates a wearable device incorporating an eye-mask incorporating a media renderer, electrodes and sensors.

FIG. 16 illustrates a rim of the eye-mask of the wearable device disposed with electrodes.

FIG. 17 illustrates rim of the eye-mask of the wearable device disposed with another configuration of electrodes and sensors.

FIG. 18 is similar to FIG. 15 devoid of a virtual reality viewer, headphones are present.

FIG. 19 is similar to FIG. 15 devoid of a virtual reality viewer and headphones.

FIG. 20 shows an exemplary visualization of an exemplary treatment session comprising four phases, as measured using LoPI.

FIG. 21 shows an exemplary visualization of a time domain response signal at a central EEG electrode during the experiment of FIG. 1.

FIG. 22 shows an exemplary visualization of a time domain response signal at a parietal EEG electrode during the experiment of FIG. 1.

FIG. 23 shows an exemplary visualization of a time domain response signal at a frontal EEG electrode during the experiment of FIG. 1.

In order to generate experimental data, according to this particular embodiment, an experiment including a hypnotic treatment session was set up. However it is clear that alternative embodiments are possible in which any other suitable treatment session is used and/or in which no use is made of a treatment session. This experiment resulted in a study with 12 healthy subjects volunteers, of which 10 were included in the subsequent analysis. The experiment comprised measurement of a level of pain by self-reporting of the subject using a validated questionnaire.

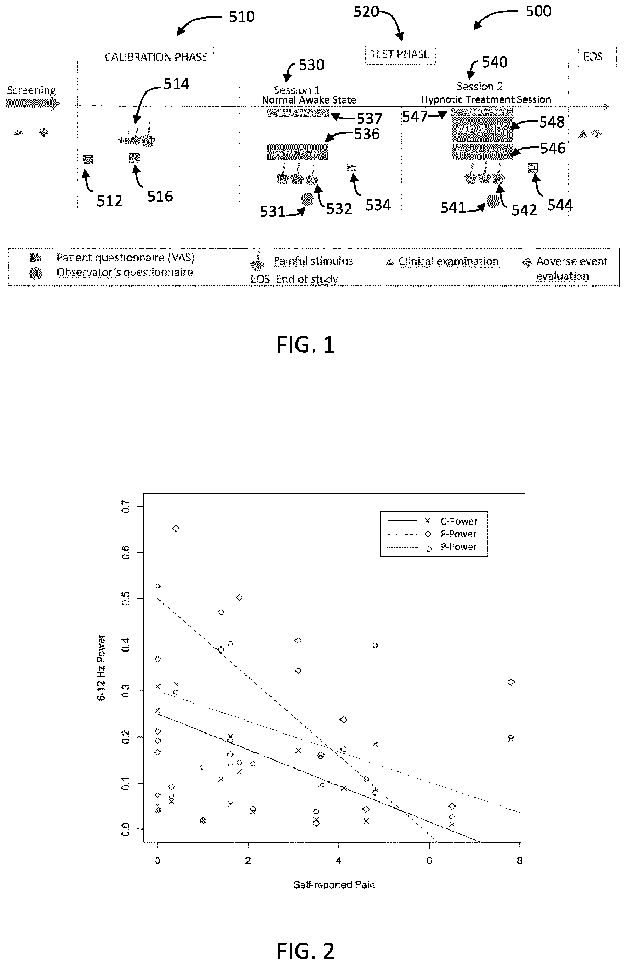

The setup of the embodiment of the experiment will be clarified by means of FIG.1 , which shows the different phases of the experiment. As shown, optionally prior to and after the different phases of the experiment clinical examination and an adverse event evaluation was performed as part of a screening and/or the end of study, EOS, in order to assure the safety of the subjects undergoing the experiment and to assure a high level of quality with respect to the self-reported data.

During an initial calibration phase 510, at a first step 512 the subjects were asked to provide self-reported values for rating parameters as explained in further detail below. Subsequently at step 514, by using a stair-case method, the subjects received a sequence of transcutaneous electrical stimulus, or in other words a painful stimulus, of increasing intensity, so that in step 516 the subject could indicate when the perceived level of pain corresponded to a level of pain with a value of 8 on a VAS pain scale. In other words, the intensity of the pain stimulus is calibrated for each subject individually using the stair-case method, by asking the subjects to indicate when they judged the perceived level of pain to corresponding to a value 8 on the pain scale, which corresponds to a level of pain qualified as "significantly painful and demanding some effort to tolerate". Such a pain scale for self-reporting of pain according to the embodiment of the experiment was determined by means of a Visual Analogue Scale or VAS, which is a measurement instrument that tries to measure a characteristic or attitude that is believed to range across a continuum of values and cannot easily be directly measured. According to this embodiment a VAS scale ranging from a value of 0 for "no pain" up to and including 10 for "severe or extreme amount of pain". Such instruments are often used in clinical research to measure the intensity of symptoms, such as for example, the level of pain as perceived by a patient. It is clear that alternative embodiments for self-reporting values are possible, such as for example the Numeric Rating Scale (NRS-11) is an 11-point scale for patient self-reporting of pain, which ranges from a value of 0 for "no pain" up to and including a value of 10 for "severe pain".

As further schematically shown in FIG.1, the calibration phase 510 was followed by a first phase 520, which was labeled as test phase 520 in Fig. 1, which comprises a first session 530 in a normal awake state of the subject. As schematically shown, during this first session 530, there were generated one or more pain stimuli, as calibrated during the previous calibration phase. As shown, according to this embodiment, there was for example generated a plurality, for example as shown in step 532 three pain stimuli that were qualified as 8 on the VAS pain scale during the calibration phase. In other words, according to this embodiment, the pain stimuli, when applied, provided for identical pain conditions, for example by means of transcutaneous electricals stimuli of identical intensity. It is however clear that alternative embodiments of the experiment are possible, in which a different number of pain stimuli and/or pain stimuli of other suitable intensities and/or varying intensities are applied. It is clear that according to this embodiment the calibrated pain stimuli 532, of a predetermined intensity, which were associated by the subject with a predetermined level of pain during the calibration phase 510, are applied at suitable times, with suitable intervals, during a time period of for example 30 minutes. However it is clear that alternative embodiments of suitable time periods, during which the one or more pain stimuli 532 are applied, are possible. During this time period, during which the pain stimuli 532 are applied, as shown at step 534, suitable sensors are configured to monitor and/or register physiological signals generated by the subject for provision to a suitable processor to generate measurement data corresponding to the state and/or activity of the body or bodily functions of the subject. Such physiological sensors provide an objective measure of physiological signals. Such physiological sensors provide for signals, that, when suitably processed, provide for measurement data based on recordings of electrical or other physiological signals produced by the brain, the heart, the muscles, the skin, ... of the subject. Embodiments of such physiological sensors are for example sensors for measuring physiological signals configured to measure respiration, electrocardiography or ECG, electromyography or EMG, Blood Pressure or BP, Blood Volume Pressure or BVP; electrooculography or EOG, electroencephalography or EEG, .... Electroencephalography or EEG is an electrophysiological monitoring method to record electrical activity of the brain. EMG measures muscle activity. EDA or skin conductance measures the hydration in the epidermis and/or dermis of the skin. EKG or ECG measures heart activity, such as for example heart rate, inter-beat-interval, heart rate variability, .... Electrooculogram measures eye movements. BVP measures blood pressure. Respiration sensors measure for example the rate of respiration, depth of breath, effort, temperature, air pressure during inhalation and/or exhalation .... According to the embodiment shown, electroencephalogram (EEG), heart rate data, electromyogram (EMG), respiratory (respiration rate, effort, temperature, and air pressure (inhalation/exhalation)) measured data were collected from each subject, however it is clear that alternative embodiments are possible comprising the monitoring of alternative electrical and/or physiological signals as described in further detail below.

Subsequent to the time period 536 during which the measurements of the physiological signals were made, the subject was asked to provide self-reported values for the level of pain, etc. for example by means a VAS patient questionnaire during step 534.

According to the embodiment of the experiment shown in FIG.1, test phase 520 subsequently comprises second session 540, which comprises an automated hypnotic treatment session 548 that was delivered using a system that comprises a virtual reality headset as will also be described in further detail below. However it is clear that alternative embodiments are possible in which any other suitable treatment session is used and/or in which no use is made of a treatment session. The embodiment of the system, also referred to as a hypnotic package, is referred to as Sedakit, as manufactured by Oncomfort. The Sedakit was configured to the Aqua 30 minutes module embodiment of an automated hypnotic treatment session, which subjected the subject to an automatic hypnotic treatment session for a time period of 30 minutes at step 548. During this hypnotic treatment session 548, similar as described above, electroencephalogram (EEG), heart rate data, electromyogram (EMG), respiratory (respiration rate, effort, temperature, and air pressure (inhalation/exhalation)) measured data were collected from each subject, however it is clear that alternative embodiments are possible comprising the monitoring of alternative physiological signals. Also similar as described above, during the hypnotic treatment session 538, each subject was exposed to one or more transcutaneous painful stimuli. For example a sequence of a plurality of pain stimuli, such as for example shown as three painful stimuli 542. Similar as described above, according to this embodiment, the painful stimuli 542 that are applied during the hypnotic treatment session 538 were qualified as 8 on the VAS pain scale during the calibration phase. In other words, according to this embodiment, the pain stimuli, when applied, provided for identical pain conditions, for example by means of transcutaneous electricals stimuli of identical intensity. It is however clear that alternative embodiments of the experiment are possible, in which a different number of pain stimuli and/or pain stimuli of other suitable intensities and/or varying intensities are applied. It is clear that according to this embodiment the calibrated pain stimuli 532, of a predetermined intensity, which were associated by the subject with a predetermined level of pain during the calibration phase 510, are applied at suitable times, with suitable intervals, during a time period of for example 30 minutes. According to the embodiment of the experiment the external pain stimulus was provided in the form of an electric transcutaneous stimulus being provided at the fore-arm during the experiment, however it is clear that alternative embodiments of a suitable pain stimulus are possible. Similarly as described above, at step 544, subsequently each subject was asked to rate pain as they perceived it during the hypnotic treatment session 548 by means of a patient questionnaire. Optionally, but preferably, during both session 1 530 in the normal awake state and session 2 540 with the hypnotic treatment session the subject is placed in a similar context. For example in both sessions, preferably the subject is placed in a similar position, for example laying on chair. As further shown, for example the subject is experiencing similar sounds 537, 547, such as for example sounds of a hospital environment. Optionally, in addition to the patient questionnaire, there may be made use of an observer's questionnaire, for example assure the quality of the experiment and/or to provide for additional and/or alternative ratings for one or more of the self-reported ratings of pain.

During the experiment, for the EEG measurements, EEG electrodes were used that were placed so as to cover the scalp and so as to detect local voltage fluctuations resulting from localized regions of the brain. According to this embodiment, a high density EEG was employed with 256 sensors or channels, however it is clear that alternative embodiments with a different number of sensors are possible. According to the embodiment of the experiment, electrodes were placed according to the predefined layout for a 256-channel Hydrocel Geodesic Sensor Network. It is further known to a person skilled in the art that EEG electrodes mainly measure neural activity that occurs at the upper layers of the brain, or in other words the neural activity of the cortex of the brain. During the experiment, for the EMG measurements sensor, a pair of surface EMG sensors were placed on a leg of each patient to as to detect the electric potential generated by muscle cells when then muscles become activated. However it is clear that alternative embodiments are possible in which EMG sensors are provided at any other suitable location, such as for example the face, arm, ... .It is however clear that according to alternative embodiments use can be made of other types of suitable EEG electrodes or sensors, EMG electrodes or sensors and/or any other suitable type of electrode or sensor for measuring EEG and/or EMG signals.

Below are the results of the above mentioned experiment, according to the embodiment of FIG.1. As mentioned above the experiment 500 comprises a test phase 520 with a "normal awake state" 530 and a "Hypnotic treatment session" for which both self-reported data with respect to the level of pain were collected in steps 538, 548 for each subject that was subjected to one or more calibrated pain stimuli. The results of the experiment of FIG.1 show measurable and statistically significant effect of the hypnotic treatment session 530 versus the normal awake state 530 regarding, the level of pain, more specifically a pain reduction as illustrated by means of Table 1 below.

Table 1 below, shows a Wilcoxon signed-rank test of the self-reported data for subjects during the experiment of FIG. 1 during the normal awake state 530 and during the hypnotic treatment session 540:

| Self-reported data 534, 544 | Normal awake state 530<br>Mean ±SD<br>Median [IQR] | Hypnotic treatment session 540<br>Mean ±SD<br>Median [IQR] | p-value | Adjusted p-value (Bonferroni correction) |
|---|---|---|---|---|
| Self-reported level of Pain | 4.21 (2.27) 4.35 [2.22] | 1.39 (1.50) 1.2 [1.65] | 0.0059 | 0.0296 |

It is thus clear that, according to the embodiment described above, in which during both the normal awake state 530 and the hypnotic treatment session 540, pain stimuli of an identical intensity were applied, or in other words, identical pain conditions were experienced by the subjects during application of the pain stimuli of identical intensity, the self-reported level of pain are significantly different in both sessions 530, 540. As for example shown, the mean of the value for self-reported pain in the normal awake state 530 is 4.21, while in the hypnotic treatment session 540 it was reported to be 1.39. In other words a reduction of more than 50% with respect to the normal awake state. It is thus clear that the self-reported level of pain corresponds to a level of pain as perceived by the subject, as clearly the self-reported values for the level of pain correlated to an identical external pain stimulus are modulated by the modified state of consciousness caused by the hypnotic treatment session. In other words, the self-reported data for the level of pain are an indicator for the level of pain as consciously perceived by the subject, or in other words the level of perceived pain.

Additionally, as shown in further detail in table 2 below, there was found to be no statistically significant correlation between the self-reported level of pain and the stimulation intensity of the pain stimulus, or in other words the intensity of the applied transcutaneous electrical stimulus. As already mentioned above, the self-reported level of pain thus corresponds to the consciously perceived level of pain perception of the subject, and is thus not directly correlated to the intensity of the external pain stimulus itself, but relates to the conscious level of pain perception. In other words the perception of pain as it appears in the conscious perception of the subject, typically associated with the cortex, after potential modulation and/or suppression by for example the thalamus and/or other downstream and/or upstream neural pathways.

Table 2 below shows the Spearman correlation for both hypnotic treatment session 540 and normal awake state 530:

|  | Self-reported level of Pain |
| --- | --- |
| Stimulation Intensity of the electrical pain stimulus | rs = -0.150 p = 0.483 |

In Table 2, the r is the general correlation coefficient. The rs is the spearman correlation coefficient. It can take a range of values from +1 to -1. A value of 0 indicates that there is no association between the two variables. A value greater than 0 indicates a positive association; that is, as the value of one variable increases, so does the value of the other variable. A value less than 0 indicates a negative association; that is, as the value of one variable increases, the value of the other variable decreases. The p is the p-value associated with the statistical test to evaluate whether the correlation is statistically significant and not just a coincidence. Generally, a p-value smaller than 0.05 is considered as statistically significant. When a p-value is closer to zero, this shows a higher statistical relevance, or in other words a better correlation.

Results from the analysis of the EEG measurement data generated during experiment 500 in combination with the self-reported data allowed associations to made between localized brain structures/networks and the perception of pain. Identification of those localized structures/networks associated with perception of pain allows for an objective monitoring of the level of pain as perceived by the subject, or in other words the level of pain perception of the subject. It is clear, as explained in further detail below, that by means of the above experiment 500, where the same calibrated external pain stimulus was applied, during a normal awake state and during a hypnotic treatment session, that the EEG measurements, which differed during the normal awake state and during the hypnotic treatment session, and which were correlated to the self-reported level of pain of the subjects, thus provide for a measurement of the a level of pain as perceived by the subject, as the calibrated external pain stimulus remained constant, and thus only the perception of the level of pain was changed during the hypnotic treatment session as compared to the normal awake state..

*Hypnotic treatment session results - measured data (EEG) - frequency domain*

In the brain, several types of brain activity occur at the same time, which is also referred to as parallel processing. Specific types of brain activity can be correlated to a specific frequency band in the measured EEG data during the experiment. In order to explore the different types of brain activity, use can be made of a suitable processing of the EEG measurement data generated during the experiment 500, by transforming the time domain signals into the frequency domain, for example by means of a fast Fourier transform or FFT operation. From such frequency domain signals, then for example the power of a specific signal frequency band can be determined, where power for example refers to a square of the amplitude of the frequency domain signals within a specified in a frequency range associated with one or more particular brain waves, such as described in further detail below. According to the embodiments described below, the frequency bands could for example comprise a combination of at least two, or exactly two types of brainwaves, such as for example a theta-alpha frequency band. This means that such theta-alpha frequency band consists of a combination of at least part of a theta frequency band and at least part of an alpha frequency band. This means that such a theta-alpha frequency band consists of a combination of at least part of a theta frequency band and at least part of an alpha frequency band.

In the EEG data of the measured data during the experiment 500, there were found significant power differences between the subject in two different experimental conditions 530, 540 associated with different levels of pain perception as reported by the subject in a frequency band comprising a combination of two different types of brain waves:

-    A power(ta) which is increased during the hypnosis treatment session 540 with respect to the normal awake state 530 in the 6-12 Hz frequency band, which is an embodiment of a theta-alpha, ta, frequency band within the theta-alpha frequency range), located around frontal lobe (F), central lobe (C) and parietal lobe (P), which is also referred to as a late component. As will be explained in further detail below, such EEG measurement data can be used as part of a group 4 indicator for the level of pain perception of a subject. It is clear from the above mentioned description that also the self-reported level of pain are also significantly different during the hypnosis treatment session 540 with respect to the normal awake state 530.

Late component refers to the power(ta) increase that was measured in a time period from 0.38 to 0.78 sec after the pain stimulus onset. According to this specific embodiment, in which there is an external pain stimulus, the power was calculated from -0.5s to 1.5s from with reference to the stimulus onset, or in other words the power was calculated in a time window of 2s including the stimulus onset. It is however clear that alternative embodiments are possible, in which for example a time window of at least 0.1s, for example a time window in the range of 0,5s up to and including 60s, or any other suitable time window is used, at any other suitable point in time with respect to the stimulus onset, as long as the time window includes at least a portion at a point in time after the stimulus onset. According to still further embodiments, such as for example during continuous power measurements, not related to a specific external stimulus, the power can be continuously calculated based on overlapping time windows of for example 2s, or of any other suitable time period, such as for example at least 0.1s, or in the range of 0.5s up to and including 60s, ...

The abovementioned results are the first identifying brain frequency band differences between an automated hypnosis treatment session and a normal awake state, especially for a brain frequency band comprising a combination of two brain waves such as for example theta and alpha brainwaves. It is clear that these results are also the first at identifying this for any other suitable treatment sessions, for example pharmacological and/or non-pharmacological treatment sessions, and/or in which no use is made of a treatment session. It is clear that this thus means that this is also the first identification of brain frequency band differences, especially a brain frequency band comprising a combination of two brain waves, with respect to different levels of pain as perceived by the subject. As already mentioned above, it is clear that such a frequency band comprising and/or consisting of a combination of two brain wave frequencies, or at least a part thereof, is different from a frequency band only comprising a single brain wave frequency band and different from a frequency band comprising more than two brain wave frequency bands.

From the measured EEG data during the experiment 500, there was extracted power(ta) at EEG electrodes F, C and/or P, and the following results were obtained:

- The self-reported measure of pain was correlated (spearman) with the computed group 4 indicator, or in other words, according to this embodiment, power(ta) at EEG electrodes F (rs = -0.29, p = 0.10), P (rs = -0.33, p = 0.15) and/or C ( rs = - 0.49, p = 0.02). Fig. 2 shows the correlation between self-reported level of pain as perceived by the subject and the computed power (ta) at EEG electrodes F, P and/or C from the measured data during the experiment 500 in more detail, for the subjects that took part in experiment 500 as described above. It is thus clear that the group 4 indicator, power (ta) is inversely correlated to the self-reported value for the level of pain;
- The group 4 indicator, or in other words power(ta) was significantly higher during the hypnosis treatment session 540 as compared to the normal awake state 530, as calculated from the EEG measurement data at the 3 EEG electrodes locations F (0.13 during normal awake state 530 vs 0.29 during the hypnosis treatment session 540, p = 0.009), P (0.16 during the normal awake state 530 vs 0.29 during the hypnosis treatment session 540, p = 0.009) and C (0.089 during normal awake state 530 vs 0.147 during the hypnosis treatment session 540, p = 0.005); This is consistent with the above-mentioned inverse correlation between the group 4 indicator, power (ta) and the self-reported value for the level of pain;

The above experimental data generated during the experiment 500, which comprises both measurement data, such as EEG data, as well as self-reported data, and which could also be referred to as response data, confirms the existence of a correlation between self-reported pain and a power, power (ta), associated with a theta-alpha frequency band, ta, which according to this embodiment extends from 6Hz up to and including 12Hz, also referred to as a group 4 indicator. However, as already mentioned above, it is clear that alternative embodiments are possible in which the frequency band comprises a combination of both theta brainwaves and alpha brainwaves. It is clear from the experimental data above that measured data received from the C, P and F EEG electrodes all show a certain level of correlation of power (ta) with the self-reported measure of pain. Although, in the current experiment the correlation is highest at the C EEG electrode, it is clear that according to alternative embodiments, the distribution of the level of the correlations amongst different EEG electrodes might be different. However, it is clear that according to alternative embodiments an alternative external pain stimulus could be provided. It is clear that instead of and/or in addition to measurement of the level of pain in reaction to an external pain stimulus, the experimental data can also be used for measurement of a level of pain caused by internal events or states of the subject, which for example could relate to pain experienced, which is not caused by an external event, such as an external pain stimulus, being applied. It is clear that alternative embodiments of the experiment are possible, in which the power(ta) is determined for any other suitable frequency band comprising a combination of both theta brain waves and alpha brainwaves, such as for example any suitable band extending in the frequency range of 4Hz up to and including 12 Hz.

These findings have been advantageously applied in a computer-implemented method for measurement of a level of pain. Based on the insight that there exists a correlation between power(ta) and self-reported pain measurements, it now becomes possible to more objectively measure a level of pain, even in cases where self-reported pain measures are not possible or available, such as for example when the subjects are in a state in which they are unable or experience difficulties to communicate, when the subjects are young children, ... It is clear that the Level of Pain, or LoP is an indicator of the level or degree to which a subject is aware and receptive to pain. In other words, it refers to the level of pain as consciously perceived by the subject. It is clear that such a level of pain perception by the subject can be modulated by for example its emotional, psychological, cognitive state, such as for example the state of consciousness of the subject. The LoP is thus an indicator for the subjective pain perception of the subject and can thus be used as an indicator of the degree to which the subject experiences pain, for example on an emotional, cognitive and/or physical level.

It is thus clear that pain in the context of this description, as for example defined by the International Association for the Study of Pain or IASP is an aversive sensory and emotional experience typically caused by, or resembling that caused by, actual or potential tissue injury. It is thus clear that pain is experienced as a conscious perception by the subject. In other words, the level of pain as experienced by the subject is a subjective experience that is influenced to varying degrees by for example biological, psychological and social factors. The conscious perception that something causes pain includes for example sensory-discriminative features, such as for example intensity, quality and location, ... and/or affective emotional aspects. It should further be noted that in this way pain as consciously perceived and nociception are different phenomena, which can occur independently of each other. The conscious experience or perception of pain cannot always be reduced to activity in sensory pathways. Nociception as defined by IASP is the neural process of encoding noxious and/or painful stimuli. The consequences of such encoding may be automatic such as for example an increased blood pressure, or behavioral, such as for example a motor reflex. However, it is clear that a conscious pain experience is not necessarily implied. As explained in further detail below, in the context of this application a Level of Pain Index, or LoPI, or Pain index, refers to a standardized index indicating the level pain of which the subject is consciously aware. In other words, the degree to which the subject consciously perceives pain at for example a sensory and/or an emotional level. This LoPI for example varies systematically with pain intensity as perceived by the subject, independently of factors not affecting pain perception. A first limit of the LoPI, for example a lower limit of the LoPI, may for example indicate that the subject experiences no conscious perception of pain. A second limit of the LoPI, for example an upper limit of the LoPI, may for example indicate that the subject experiences the strongest/worst possible conscious perception of pain.

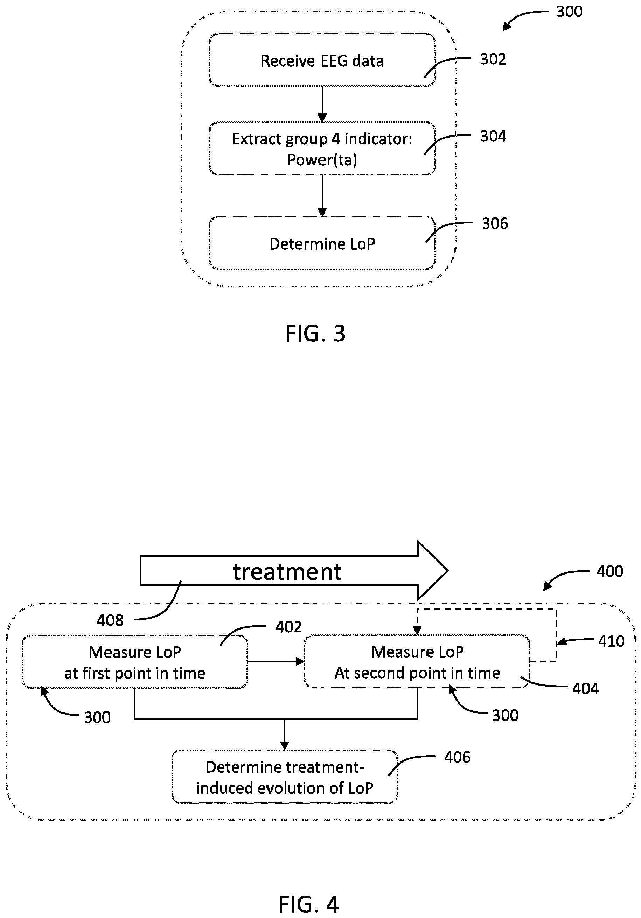

According to the embodiment shown in FIG. 3, such a computer-implemented method for an objective measurement of a level of pain 300 as consciously perceived by a subject, for example comprises the steps of receiving EEG data 302, extracting power(ta) 304 and determining a level of pain, LoP, 306. At step 302 there is received measured data comprising electroencephalogram, EEG, data collected from one or more EEG electrodes. At step 304, there is then extracted from the EEG data, a group 4 indicator corresponding to: a power, power (ta), associated with a theta-alpha frequency band, ta, within a theta-alpha frequency range. At step 306 there is then determined, based on said group 4 indicator, a level of pain, LoP, which is a value indicative for the level of pain of the subject. It is clear that, according to alternative embodiments, optionally, in addition to said group 4 indicator, further elements, such as other group indicators, measurements, parameters, reference measurements, ... could be taken into account for determining the LoP. It is clear that according to particular embodiments the method could for example comprise additional steps, such as for example after the step of receiving EEG data or any other suitable data, pre-processing the EEG data or other data, subsequently extracting relevant features, thereby allowing the determination of for example group 4 indicators or other suitable group indicators, and subsequently normalization and/or classification, thereby for example determining LoP or an indexed and/or scaled version of LoP, such as for example LoPI as will be explained in further detail below. It is however clear that still further alternative embodiments are possible.

According to the embodiment of the experimental data generated above, it was found that for a population with a mean self-reported pain value of 4.2, there was measured a mean power(ta) of 0.16 mV$^2$, when a calibrated pain stimulus of value 8 on the pain scale was applied in normal awake state 530. For a population with a mean self-reported pain value of 1.4, there was measured a mean power (ta) of 0.29 mV$^2$, when a calibrated pain stimulus of value 8 on the pain scale was applied during the hypnotic treatment session 540. As already mentioned above, from the experimental data above, the LoP is inversely correlated to the group 4 indicator, power(ta), or in other words an increase in power(ta) corresponds to a decrease in the LoP, and a decrease in power(ta) corresponds to an increase in the LoP as consciously perceived by the subject. As already mentioned above, it is clear that the power(ta) values mentioned above, were calculated from -0.5s to 1.5s from with reference to the stimulus onset, or in other words the power was calculated in a time window of 2s including the stimulus onset, according to an embodiment where there was an external pain stimulus. As further mentioned above, it is however clear that alternative embodiments are possible, in which alternative suitable time windows are being used, and/or in which the time window is not related to a specific external stimulus, such that for example the power can be continuously calculated based on overlapping time windows.

According to FIG. 4, this computer-implemented measurement of the LoP based on measurement data could be applied to determine and monitor a treatment induced evolution of the LoP. When for example a treatment is applied, such as for example a pain reducing treatment, or any other suitable treatment, the effect of this treatment on the LoP may be monitored by the present method. Such treatments might for example comprise any suitable treatment, such as non-pharmacological treatments, such as for example non-pharmacological sedation, hypnosis, other evidence-based psychological and/or mind/body interventions, etc. , and/or pharmacological treatments, such as for example pharmacological sedation, a treatment comprising administration of active pharmacological ingredient, administration of an analgesic, and/or any other suitable treatments such as for example medical or paramedical treatment such as surgery, physical therapy, electrical treatment, mechanical treatment, ... , and/or mind/body complementary or alternative medicines such as acupuncture, biofeedback, massage.... In this way FIG. 4 shows an embodiment of a computer-implemented method for determining and/or monitoring a level of pain in a subject before, during and/or after a treatment 400. The embodiment of the method 400 shown in FIG. 4, comprises the steps of:

- measuring the LoP at a first point in time 402, for example before the treatment or at a first point in time during the treatment;
- measuring the LoP at a second point in time 404, for example after the treatment, or at a second point in time during the treatment later than the first point in time; and
- determining the treatment-induced evolution of the LoP 406.

The treatment, and its associated chronology, has been schematically illustrated by means of arrow 408 in the embodiment of FIG. 4, which could for example represent a singular treatment session with a well-defined start and end-point, however it is clear that alternative embodiments are possible in which the treatment for example has continuous and/or chronic nature without a well-defined start and end-point, or in which the treatment has an intermittent nature and comprises a sequence of a plurality of treatment sessions with optional breaks or pauses in between these treatment sessions, or any other suitable type of treatment comprising a chronological nature, for example comprising a sequence of treatment phases, steps, etc.

As shown at step 402 the LoP is measured, for example with the method for measurement of LoP 300 according to the embodiment shown in FIG. 3. This first measurement of LoP could for example be a measurement of the LoP before the start of a treatment session and could, according to some embodiments be considered as a reference, initial, baseline, ... LoP that serves as a predetermined reference level for subsequent measurements of LoP. As further shown in FIG. 4, at step 404, the second, subsequent measurement of LoP, is for example also measured with the method for measurement of LoP 300 shown in FIG. 3. This could for example be a measurement of the LoP during or after the treatment, for example a pain reducing treatment. At step 406 then there is determined the treatment-induced evolution of the LoP, based on a comparison of the first LoP and second LoP, measured at steps 402 and 404 respectively. In this way the treatment-induced evolution of the LoP is be able to show a value representative for the effectiveness, intensity, desired effect, etc. of the treatment. If the treatment, for example is a pain reducing treatment, then a treatment-induced evolution of the LoP that corresponds to a reduction of the second LoP measured at step 304 as compared to the first LoP measured at step 302, is able to confirm the effectiveness of the treatment. According to some embodiments the value of the treatment-induced evolution of the LoP also provides for an indication of the intensity of the treatment. For example, according to an embodiment, a pain reducing treatment reduces a first LoP measurement with a first value at step 402 to second value, that corresponds to a 50% reduction of the first value, at a second measurement at step 404, which thus corresponds to a treatment-induced evolution of the LoP of -50% or in other words a change in the LoP corresponding to a reduction of 50% with respect to the measurement at step 302 before the treatment. When this treatment is adapted or another treatment is applied, which only causes a treatment induced evolution of the LoP of -25%, it is clear that this latter treatment has an intensity that is lower than the former. When no change is detected with respect to the first LoP measurement, it can be concluded that the treatment-induced evolution of the LoP corresponds to no change to the LoP, or in other words, that the treatment at that point in time is for example not effective in reducing the LoP and might need adaptation or change to another type of treatment. However, according to alternative embodiments, in which for example the treatment comprises the application of painful stimuli, such as for example during a surgical treatment, when the LoP as measured before the treatment, does not change, does not increase and/or does not increase above a predetermined threshold, during the treatment, especially during and/or after the application of the painful stimuli, it is clear that the pain treatment, for example in the form of pharmacological and/or non-pharmacological sedation is performing effectively.

As schematically shown by means of striped line 410, according to an optional embodiment, the measurement of a further LoP at step 404, at a later point in time than at step 402, could be repeated or could be performed continuously, which allows for a repeated and/or continuous monitoring of the treatment-induced evolution of the LoP, or in other words the comparison of two LoP measured at two different points in time. It is clear that alternative embodiments are possible in which any suitable plurality of LoP measurements at different points in time, for example before, during and/or after the treatment, are used in order to determine and/or monitor an evolution, for example a treatment-induced evolution, of the LoP, based on a comparison of these at least two LoP measured at at least two different points in time. It is thus clear that such a treatment induced evolution of the LoP may be indicative of a treatment induced change, if any, to the LoP. However, it is clear that alternative embodiments are possible in which the LoP is measured at at least two different points in time during any suitable time period for determination of any suitable evolution of the measured LoP.

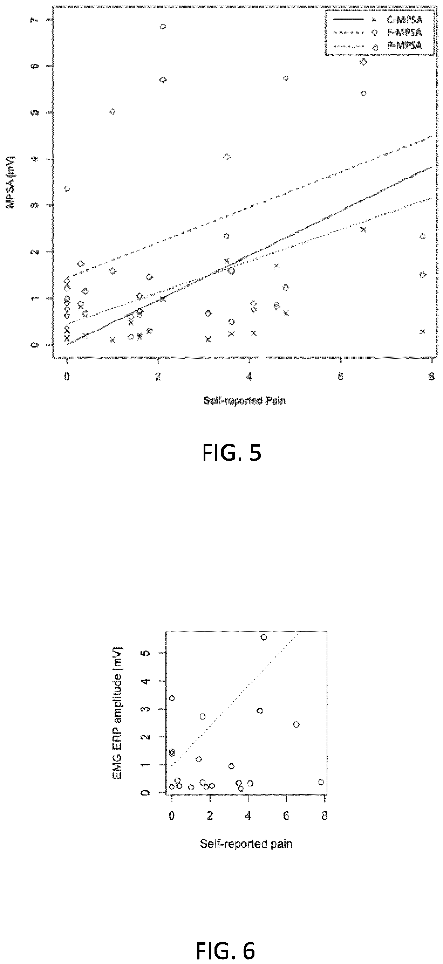

In addition to the group 4 indicator mentioned above with respect to the experiment 500 as shown in FIG.1 and the application of the results of this experiment in the computer-implemented methods of Figures 4 and 5, the LoP can optionally be determined by means of additional indicators, such as for example a group 3 indicator or a group 5 indicator as described in further detail below.

According to such an embodiment, during the embodiment of the experiment as shown in FIG.1, in addition to the group 4 indicator, there is for example extracted from the measured EEG data a group 3 indicator in the time domain, for example by means of a suitable event related potential, or ERP, in response to the calibrated pain stimulus.

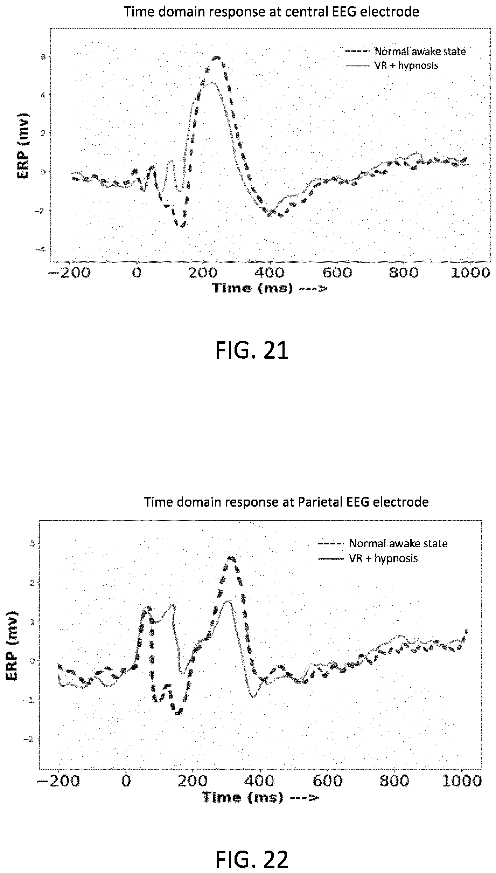

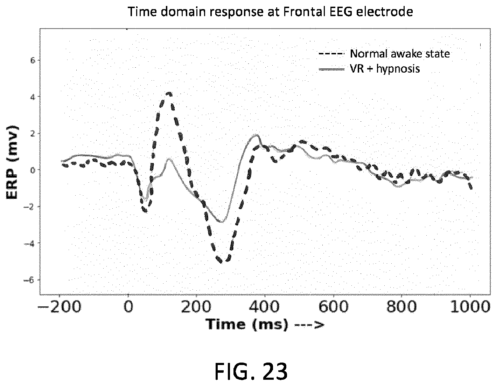

Such a time-domain EEG analysis showed an overall decreased in the ERP components during the hypnotic treatment session 540 as compare to the normal awake state 530. Based on the measured EEG data, there was found a difference in overall mean signal peak-to-peak amplitude, or MSPA, time-locked to the painful stimulus onset. According to a particular embodiment, as shown, in FIG. 21, 22 and 23, the time window could for example be set from 300ms to 350ms after the painful stimulus onset, which is represented as 0ms on the x-axis. However it is clear that alternative embodiments are possible in which the MSPA is measured on an ongoing basis, for example by means of a suitable, rolling time window, not linked or triggered by an external pain stimulus. In case there is only a single peak in the time window for measurement of the MSPA, the MSPA is equal to the peak-to-peak amplitude of the signal. In case there are multiple peaks in the signal in the time window for measurement of the MSPA, the MSPA will be calculated in function of the mean, average, weighted mean, ... of the individual peak-to-peak amplitudes of the plurality of these peaks of the signals. The difference in this embodiment of the ERP amplitude between the 2 sessions 530, 540 of the first phase 520, in other words between the normal awake state 530 and the hypnotic treatment session 540 that induces a different level of perceived pain, was found to be significant at least at the following 3 EEG measurement locations:

- at an EEG electrode positioned at the central lobe (C), for example specifically along the midsagittal plane: there was found a decrease in overall MSPA during the hypnotic treatment session 540 as compared to the normal awake state 530, in other words the C-MSPA EEG measurement data shows a decreased involvement in the response to the calibrated pain stimulus;
- at an EEG electrode positioned at the parietal lobe (P), for example specifically along the midsagittal plane: there was found a decrease in overall MSPA during the hypnotic treatment session 540 as compared to the normal awake state 530, in other words the P-MSPA EEG measurement data shows a decreased involvement in the response to the calibrated pain stimulus;
- at an EEG electrode positioned at frontal lobe (F), for example specifically along the midsagittal plane: there was found a decrease in overall MSPA during the hypnotic treatment session 540 as compared to the normal awake state 530, in other words the P-MSPA EEG measurement data shows a decreased involvement in the response to the calibrated pain stimulus.

The study based on this experiment is thus showing statistically significant MSPA changes at those specific locations of the EEG measurement data, when comparing in two different experimental conditions 530, 540 associated with different levels of pain perception as reported by the subject and which correlate to the different level of pain perceived in reaction to a calibrated painful stimulus.

The extraction of the mean signal peak-to-peak amplitude, or MSPA, for electrodes F, C and/or P, from the EEG data of the measurement data of the experiment 500, which can also be referred to as group 3 indicators, provided the following results:

- The MSPA extracted from the EEG data, or the group 3 indicator, is significantly lower during the hypnotic treatment session 540 as compared to the normal awake state 530 at at least the following 3 EEG electrode locations: at EEG electrode location F there was extracted a F-MSPA value of 24.13 mV during the normal awake state 530 versus an extracted a F-MSPA value of 11.16 mV during the hypnotic treatment session, p-value = 0.019, at EEG electrode location C there was extracted a C-MSPA value of 8.67 mV during the normal awake state 530 versus an extracted a C-MSPA value of 2.94 mV during the hypnotic treatment session 540, p-value = 0.037, and at EEG electrode location P there was extracted a P-MSPA value of 5.159 mV during the normal awake state 530 versus an extracted a P-MSPA value of 2.635 mV during the hypnotic treatment session 540, p-value = 0.037. Similar as above, it is thus clear that the group 3 indicator is an indicator for the level of pain as consciously perceived by the subject, as the group 3 indicator is modified by a change of the state of consciousness under identical pain conditions of the experiment, in which an identical calibrated pain stimulus was applied as described above.
- The self-reported measure of pain is correlated (spearman) with the group 3 indicator, or in other words the MSPA extracted from the EEG measurement data, at at least the following 3 EEG electrode locations: F (rs = 0.38, p = 0.097), C (rs = 0.407, p = 0.074) and/or P (rs = 0.34, p = 0.095). FIG. 5 shows a graph indicating the correlation between the group 3 indicator, or in other words the MSPA extracted from the measured EEG data at the 3 EEG electrodes F, C and P and pain during the experiment 500.

As already mentioned above in relation to the signals shown in FIG. 21, 22 and 23, in case there is only a single peak in the time window for measurement of the MSPA, the MSPA is equal to the peak-to-peak amplitude of the signal. In case there are multiple peaks in the signal in the time window for measurement of the MSPA, the MSPA will be calculated in function of the mean, average, weighted mean, etc. of the individual peak-to-peak amplitudes of the plurality of these peaks of the signals. The time window for measurement could for example be in the range of 0.01s to 10s, such as for example in the range of 0.1s to 5s.

The study of the experiment 500 mentioned above, thus shows that significant MSPA changes in the measured EEG data have been observed when comparing different levels of pain as perceived by the subject, as the level of pain perceived by the subject during the normal awake state 530 differed from the level of pain perceived by the subject during the hypnotic treatment session 540.

Similar as described above, the insights of the experiment 500 above allow for an alternative embodiment of a computer-implemented method 300 for measurement of a level of pain comprising the further step of extracting from the EEG data, a group 3 indicator corresponding to a mean signal peak-to-peak amplitude, MSPA. Then at a subsequent step the LoP is determined based on the group 4 indicator and at least the group 3 indicator. It is clear that similarly an alternative embodiment of the computer-implemented method 400 for determining and/or monitoring a level of pain of a subject, comprising this alternative method for measurement of a level of pain is possible.

According to still a further embodiment there is provided a computer-implemented method 300 for measurement of a level of pain comprising the further step of receiving measured data comprising electromyography, EMG, data, for example collected from one or more EMG electrodes or any other suitable electrode. These EMG electrodes could for example be positioned at one or more of the following positions: a leg, face, arm, or any other suitable position, .... According to still further embodiments it is possible to derive EMG signals and/or EMG data from signals and/or data received from any other suitable electrodes, such as for example EEG electrodes, etc. During a subsequent step extracting from the EMG data, a group 5 indicator corresponding to a mean signal peak-to-peak amplitude, EMG-MSPA. Subsequently then determining the LoP based on the group 4 indicator and at least the group 5 indicator.

Such an embodiment is made possible by means of the following insights resulting from the embodiment of the experiment 500 mentioned above, in which EMG measurement data resulted from suitable measurement of EMG signals during the test phase 520. There was found a decreased MSPA of the ERP-EMG triggered by the pain stimulus during the hypnotic treatment session 540 as compared to the normal awake state 530. There was also found a correlation between the ERP-EMG triggered by the pain stimuli between the EMG-MSPA and the self-reported level of pain perception ($r= 0.387$, p-value = 0.09). It was further found that a wider ERP-EMG was associated with an increased self-reported level of pain perception. FIG. 6 shows the correlation between the self-reported level of pain perception and the EMG-ERP MSPA extracted from the EMG measurement data during the experiment 500.

According to a preferred embodiment of the method 300 the LoP is determined based on the group 4 indicator, the group 3 indicator, and the group 5 indicator.

Similar as described above, according to a preferred embodiment, the method for measuring the LoP 300 comprises the step of receiving the measured data of the subject comprising the electroencephalogram, EEG, data collected from one or more of the following EEG electrodes:

- a frontal (F) EEG electrode configured for collection of F-EEG electrode data from the scalp anatomical region corresponding to a frontal lobe of the subject,
- a parietal (P) EEG electrode configured for collection of P-EEG electrode data from the scalp anatomical region corresponding to a parietal lobe of the subject,
- a central (C) EEG electrode configured for collection of C-EEG electrode data from the scalp anatomical region corresponding to a precentral and postcentral gyrus of the subject.

Such EEG data is related to brain waves generated by parts of the brain close to the scalp, including the cortex, and therefor are related to the conscious perception of the level of pain by the subject.

Electroencephalography (EEG) is a technique well known in the art for recording electrical activity of the brain. EEG Electrodes placed along the scalp at certain locations measure voltage fluctuations resulting from electrical impulses within the neurons of the brain, mainly in the region of the brain close to the scalp, including the cortex. EEG measurement data reflects how different neurons/ populations of neurons in the brain networks communicate with each other by means of electrical impulses. Electroencephalography produces an electroencephalogram. The electroencephalogram, which thus forms the EEG data, may be refined to a particular electrode location, such as the frontal, parietal or central electrodes mentioned above. From the EEG data there may for example be extracted a frequency-based parameter and/or a phase-based parameter, and/or amplitude-based parameter. Frequency-based parameters may for example be obtained by a frequency-based analysis of EEG measurement data. Such a frequency based analysis of EEG measurement data may for example be used to measure the power contained in a specific signal frequency band, where power refers to a square of amplitude of the frequency domain signals typically within a specified in a frequency band or range, such as for example a frequency band spanning or within a delta-theta frequency range, or a frequency band spanning or within a theta-alpha frequency range.

The "theta-alpha" (ta) frequency band refers to a band of frequencies in a frequency range encompassing both theta and alpha brain waves. The theta-alpha (ta) frequency range is typically greater than or equal to 4 Hz and equal to or less than 12 Hz. The theta brain waves in this theta-alpha frequency band are preferably fast theta brain waves, for example greater than 6 Hz and equal to or less than 8 Hz. The width of the theta-alpha (ta) frequency band may be at least 2 Hz. Most preferably the theta-alpha (ta) frequency band is greater than 6 Hz and less than or equal to 12 Hz. In other words the theta-alpha (ta) frequency band comprises and preferably consists of a combination of at least part of the theta frequency band and at least part of the alpha frequency band.

An amplitude-based parameter may be obtained by amplitude -based analysis of EEG measurement data, such as for example a mean signal peak-to-peak amplitude or MSPA in a time domain. The signal peak amplitude is the maximum excursion of the signal wave from the zero point. The signal peak-to-peak amplitude is the distance from a negative peak to a positive peak.

The EEG data may be acquired with one or more EEG electrodes placed on the scalp in the anatomical regions corresponding to the position of the frontal lobe, the parietal lobe and/or both precentral and postcentral gyrus.

A frontal EEG electrode or F-EEG electrode is located on the scalp anatomical region corresponding to the frontal lobe. It is configured for attachment to or contact with the scalp anatomical region corresponding to the frontal lobe. It records F-EEG electrode data. The frontal lobe refers to the anatomical part of the brain. The region of the frontal lobe may be along the sagittal plane, more preferable to in middle of the scalp anatomical region corresponding to the frontal lobe, along the midsagittal plane

A parietal EEG electrode or P-EEG electrode is located on the scalp anatomical region corresponding to the parietal lobe. It is configured for attachment to or contact with the scalp anatomical region corresponding to the parietal lobe. It records P-EEG electrode data. The parietal lobe refers to the anatomical part of the brain. The region of the parietal lobe may be along the sagittal plane, more preferable to in middle of the scalp anatomical region corresponding to the parietal lobe, along the midsagittal plane

A central EEG electrode or C-EEG electrode is located on the scalp anatomical region corresponding to the precentral and postcentral gyrus. It is configured for attachment to or contact with the scalp anatomical region corresponding to the precentral and postcentral gyrus. It records C-EEG electrode data. The precentral and postcentral gyrus refers to the anatomical part of the brain. The region of the precentral and postcentral gyrus may be along the sagittal plane, more preferable to in middle of the scalp anatomical region corresponding to the precentral and postcentral gyrus, along the midsagittal plane.

It is clear that alternative embodiments are possible in which in one or more EEG electrodes are arranged at the above positions, and or additional suitable scalp anatomical regions. As a minimum one localized EEG electrode may be used, such as for example positioned at the scalp anatomical region of the frontal lobe. However, it is understood that a second electrode, or ground electrode may be employed for common mode rejection, i.e. to dissociate relevant information from noise; this can be placed anywhere on the scalp, for instance in another region of the scalp, in a bilateral position. A third electrode, or reference electrode, may be employed to compute voltage differences. This third electrode can be placed anywhere on the scalp, for instance at an electrically neutral location.

It is thus clear that the measured data may comprise one or more measured data components, such as for example EEG data, which is a measured data component, EMG data, ....

In addition to the measured data, the embodiments of the experiment 500, method for measurement of the LoP 300, etc. may in addition to measured data optionally also take into account observational data and/or self-reported data. In other words these methods may make use of response data that may comprise measured data and optionally observational data and/or self-reported data. The response data may comprise measured data and/or observational data and/or self-reported data. The response data contains one or more data components, each component being derived from a single measurement (e.g. EEG, EMG, EDA, ECG, EOG), single observation (e.g. movement, skin color), or single self-reported event such as for example a self-reported level of pain as mentioned above.

It is clear that the measured data is data obtained from the subject using one or more devices such as an electrode and/or sensor, such as for example a transducer, camera, motion sensor, ... disposed in relation to the subject. Observational data is then data obtained from a non-self, or hetero, observation of the subject. Self-reported data is data or information reported by the subject, for example before, during or after the measurement of the LoP and/or the treatment.

According to particular embodiments the response data may comprise electrical activity data. Electrical activity data is measured data. Electrical activity data is any data derived from a measurement obtained by an electrical electrode. The electrode is typically placed on the skin. Usually the data is captured by at least 2 electrodes (e.g. 2, 3, 4, 5 or more). Examples of electrode-captured data include electroencephalogram (EEG) data, electromyography (EMG) data, electrodermal activity (EDA) data, galvanic skin response (GSR), electrocardiogram (ECG) data, electrooculogram (EOG) data. It is preferred that the response data comprises at least EEG data. Any electrical data component (e.g. EEG data) may or may not be processed prior to being used as measured data and/or response data.

According to particular embodiments the response data may comprise physiological data. Physiological data is measured data. Physiological data is data derived from a measurement on the subject obtained by a device usually containing a transducer, camera, motion sensor. Physiological data excludes the above-mentioned electrical activity data. Examples of physiological data include pulse rate data, heart rate data (e.g. heart rate, heart rate variation data), blood pressure data, respiratory (rate) data (e.g. respiratory rate, respiratory rate variation data), brain oxygenation data, blood O2 saturation data, regional and/or central blood O2 saturation data, body temperature data, photoplethysmogram (PPG) data. Blood oxygen saturation may be, for instance SpO2, SvO2, and the like. Any physiological data component (e.g. pulse rate data) may or may not be processed prior to being used as response data. It is clear that, according to some embodiments, physiological data could be derived from electrical activity data, such as for example heart rate data could for example be derived from ECG data, etc.

According to exemplary embodiments in which the response data further comprises ECG and/or heart rate data and/or breathing rate data, the LoP can further be determined in function of such response data, as during the experiment it was detected that:

- Heart rate variability was significantly higher during painful stimulation (mean 137 ms, standard deviation 64) as compared to rest (mean 97 ms, standard deviation 10) (p-value = 0.017); and
- Breathing rate was significantly lower during painful stimulation (mean 9.8 cycles/sec, standard dev 1.2) as compared to rest (mean 11.06 cycles/sec, standard deviation 1.16) (p-value = 0.032).

According to particular embodiments the response data may comprise motion-tracking data. Motion-tracking data is measured data. The motion tracking data is data derived from a movement of the body or of a part of the body. Typically, motion tracking data is measured by one or more motion sensors (e.g. a 2- or 3-axis accelerometer, gyroscope, one or more cameras). The motion-tracking data may comprise body one or more of body tracking data (e.g. head, limb (arms legs hands), bodily shaking) and/or eye tracking data. The motion may be spontaneous and/or as a result of a stimulation.

According to particular embodiments the response data may comprise facial expression data. Facial-expression data is measured data. The facial expression data is data relating an emotion and/or pain perception. Typically, facial expression data is measured by one or more cameras directed at the face (or EMG sensors).

According to some embodiments Heart rate data and/or respiratory data could also be derived from motion tracking data and/or data generated by one or more cameras directed at the face.

According to particular embodiments the response data may comprise observational data. The observational data is data observed about the subject by another person. The observational data is data observed by the user (e.g. care provider such as a physician, or medical assistant, or non-medical assistant (e.g. friend, relative, helper)), or by a stakeholder associated with the user (e.g. hospital), or obtained from a database (e.g. medical records). The observed data may comprise one or more observed data components (e.g. a subject's movements is an observed data component). Observational data may include one or more of subject's movements/ lack of movement; procedural events; clinical observation (skin color, groaning or verbalization of discomfort...); age, type of surgery, ethnic origin, language; dosages of sedation drugs.

According to particular embodiments the response data may comprise self-reported data. The self-reported data is data reported by the subject. The self-reported data may for example be provided during a treatment session. Preferably, the self-reported data is provided before, during and/or after the treatment session. The self-reported data may comprise one or more self-reported data components (e.g. level of pain during measurement, or during treatment is a self-reported data component). Self-reported data may include a self-reported level of pain for example before, during and/or after treatment, estimated duration of the procedure, recall of the events during the session, ....

According to embodiments comprising EMG measured data, EMG data may be acquired using two or more EMG electrodes placed on the skin, preferably on limbs or face; or derived from another sensor like EEG. It represents electrical activity data from the subject muscle tissue. It may comprise a frequency-based parameter and/or a phase-based parameter and/or an amplitude-based parameter. However, it is clear that according to alternative embodiments EMG measured data, might for example be extracted from any suitable electrodes, such as for example from signals measured by EEG electrodes.

According to further embodiments the following measured data may optionally and/or additionally be captured and processed during alternative embodiments of the methods described above and/or below.

Heart rate measurement data may be acquired using two or more electrocardiogram (ECG) electrodes placed on the skin using peripheral or precordial placement, or using any other placement where ECG data can be derived. ECG detects electrical activity of the heart. Alternatively or in addition, heart rate data may be acquired using a photoplethysmogram (PPG) sensor placed on the skin. PPG detects blood volume changes vasculature below the skin. Heart rate data may comprise heart rate, heart rate variability, derived metrics, phase-based parameters, frequency-based parameters. However, it is clear that according to alternative embodiments ECG measured data, might for example be extracted from any suitable electrodes, such as for example from signals measured by EEG electrodes.

Respiratory measurement data may be acquired using one or more force/movement transducer (e.g. chest band) or air pressure sensor, or derived from a PPG sensor or from ECG electrodes placed at location where respiratory rate is detectable. The respiratory data may comprise one or more of respiration rate, respiration rate variability (also known as respiration variability), inhalation pressure, exhalation pressure, respiratory rhythm, respiratory depth, respiratory pattern and derived indexes. Changes of respiration patterns may for example also indicate changes to the LoP. Respiratory data may be known herein as respiratory rate data. However, it is clear that according to alternative embodiments Respiratory measurement data, might for example be extracted from any suitable sensors, such as for example from signals measured by a 2- or 3-axis accelerometer or a gyroscope.

The response data according to a particular embodiment may comprise measured data that comprises EEG data, and optionally EMG data, ECG data, and eye tracking data, ... , observational data that comprises patient's observed movement or lack of movement and dosages of received medication and/or self-reported data that comprises self-reported consciously perceived level of pain ratings, .... Preferably the response data comprises EEG data. Preferably the self-reported data (if any) comprises ratings for a level of pain.

Preferably the measured data comprises EEG data that comprises data collected from the F-EEG electrode and/or P-EEG electrode and/or C-EEG electrode, and the group 4 indicator is determined by:

- extracting from the F- EEG electrode data, a power associated with a band in the theta-alpha, ta, frequency range (F-power (ta)); and/or
- extracting from the P- EEG electrode data, a power associated with a band in the theta-alpha, ta, frequency range (P-power (ta)); and/or
- extracting from the C- EEG electrode data, a power associated with a band in the theta-alpha, ta, frequency range (C-power (ta));

wherein the F-power (ta) and/or P-power (ta) and/or C-power (ta) (Group 4 indicators) are a measurement indicative for the LoP as perceived by a subject.

The embodiments of the Group 4 indicators:

- one or more of F-power (ta), P-power (ta), C-power (ta)

are indicative of the level of pain the subject consciously perceives.

Preferably the EEG data comprises data collected from the C-EEG electrode and optionally from P-EEG electrode and optionally from the F-EEG electrode, and the determining comprises:

- extracting from the C-EEG electrode data, the C-power (ta),
- optionally extracting from the P- EEG electrode data, the P-power (ta), and
- optionally extracting from the F-EEG electrode data, the F-power (ta)

wherein the C-power (ta), and optionally P- power (ta) and optionally F- power (ta) are indicative of the LoP.

Preferably the EEG data comprises data collected from the C-EEG electrode and from P-EEG electrode and optionally from the F-EEG electrode, and the determining comprises:

- extracting from the C-EEG electrode data, the C-power (ta),
- extracting from the P- EEG electrode data, the P-power (ta), and
- optionally extracting from the F-EEG electrode data, the the F-power (ta)

wherein the C-power (ta) and P- power (ta) and optionally F- power (ta) are indicative of the LoP.

The EEG data may comprise data collected from the C-EEG electrode and the P-EEG electrode and the F-EEG electrode, and the determining comprises:

- extracting from the C-EEG electrode data, the C- power (ta), and
- extracting from the P- EEG electrode data, the P- power (ta), and
- extracting from the F-EEG electrode data, the F- power (ta),

wherein the combination of C- power (ta) and P-power (ta) and F- power (ta) are indicative of the LoP.

It is clear that alternative embodiments of the EEG data collected from any suitable combination of one or more of the above mentioned EEG electrodes, and/or at least one other EEG electrode are possible. It is thus clear that still further embodiments of the group 4 indicator are possible in which EEG electrode data from one or more electrodes at any suitable scalp location is used to extract a power (ta) or in other words a power associated with a theta-alpha frequency band that is indicative for the LoP consciously experienced by the subject.

According to a particular embodiment a value of power (ta) compared with a reference value is further indicative of the LoP experienced by a subject. For example, a value of said F-power (ta) compared with a reference value is further indicative of the LoP experienced by a subject, a value of said P-power (ta) compared with a reference value is further indicative of the LoP experienced by a subject, and/or a value of said C-power (ta) compared with a reference value is further indicative of the LoP experienced by a subject.

According to such an embodiment an increase of power(ta), for example of F-power (ta) and/or P-power (ta) and/or C-power (ta) compared with a respective reference value(s) is indicative of a reduced LoP and vice versa.

As will be explained in further detail below such reference values, or reference data can be used to scale and/or index the measured data, such as for example power(ta) or any other measured data and/or the parameters determined from it, such as for example the LoP. According to such embodiments reference data comprising measured data of a subject and/or a population correlated to at least one predetermined reference LoP is received. This could for example be reference data generated by the subject itself, such as a process similar as the calibration phase or the normal awake state of the experiment, for example prior to a treatment session. According to alternative embodiments the reference data and the correlations could result from separate and distinct subject or population based reference measurements. There can then be determined at least one correlation of at least one predetermined reference LoP and at least one group indicator extracted from the reference data. In this way, similar as described above, for example one or more particular values of the group 4 indicator power(ta) can be correlated with one or more corresponding particular reference values for LoP. These correlations then allow to scale and/or index any subsequently measurements of such a group indicator, such as for example the group 4 indicator power(ta). It is clear that similarly for other group indicators there can be scaled and/or indexed a subsequently measured LoP with respect to the at least one predetermined reference LoP by means of said at least one correlation.

According to a particular embodiment, similar as already mentioned above, the LoP is also based on a group 3 indicator. According to such an embodiment, preferably the EEG data comprises data collected from the C-EEG electrode, and/or P-EEG electrode and/or F-EEG electrode, and the determining comprises:

- extracting from the C- EEG electrode data, a mean signal peak-to-peak amplitude (C-MSPA), and/or
- extracting from the P-EEG electrode data, a mean signal peak-to-peak amplitude (P-MSPA), and/or
- extracting from the F-EEG electrode data, a mean signal peak-to-peak amplitude (F-MSPA),

wherein the C-MSPA, and/or P-MSPA and/or F-MSPA, embodiments of Group 3 indicators, are indicative of the LoP of a subject. In particular, they are indicative of how much pain the subject consciously perceives.

The embodiments of Group 3 indicators:

- one or more of C-MSPA, P-MSPA, F-MSPA

are indicators of the LoP of a subject.

Similar as explained above with reference to the group 4 indicator, also for the group 3 indicator there can be made use of reference data to scale and/or index the measured data for determining the LoP. For example, a change of said C-MSPA and/or P-MSPA and/or F-MSPA compared with a respective reference value(s) is indicative of a change to the LoP of the subject. In particular a reduction of said C-MSPA and/or of said P-MSPA and/or of said F-MSPA is indicative of a decrease in the LoP of the subject and an increase with respect to the reference value is thus indicative for an increase in the LoP. It is clear that similar as described above with respect to group 4 indicator, also for group 3 indicator, and/or any suitable combination of group indicators and/or other group indicators, there can be scaled and/or indexed a subsequently measured LoP with respect to the at least one predetermined reference LoP by means of said at least one correlation, which could be subject based or population based.

According to a preferred embodiment the EEG data comprises data collected from the C-EEG electrode and optionally from P-EEG electrode and optionally from the F-EEG electrode, and the determining comprises:

- extracting from the C-EEG electrode data, the C-MSPA,
- optionally extracting from the P- EEG electrode data, the P-MSPA, and
- optionally extracting from the F-EEG electrode data, the F-MSPA

wherein the C-MSPA, and optionally P-MSPA and optionally F-MSPA are indicative of the LoP of a subject.

According a further embodiment the EEG data may comprise data collected from the C-EEG electrode and from P-EEG electrode and optionally from the F-EEG electrode, and the determining comprises:

- extracting from the C-EEG electrode data, the C-MSPA, and
- extracting from the P- EEG electrode data, the P-MSPA, and
- optionally extracting from the F-EEG electrode data, the F-MSPA

wherein the C-MSPA, and P-MSPA and optionally F-MSPA are indicative of the LoP of a subject.

According to a further embodiment the EEG data may comprise data collected from the C-EEG electrode and the P-EEG electrode and the F-EEG electrode, and the determining comprises:

- extracting from the C-EEG electrode data, the C-MSPA, and
- extracting from the P- EEG electrode data, the P-MSPA, and
- extracting from the F-EEG electrode data, the F-MSPA

wherein the combination of C-MSPA, and P-MSPA and F-MSPA are indicative of the LoP of a subject.

According to a particular embodiment, similar as already mentioned above, the LoP is also based on a group 5 indicator. According to such an embodiment, preferably the measured data may further comprise electromyography, EMG, data, for example collected from one or more EMG electrodes, or derived from any other suitable electrode, such as for example EEG electrodes. There can be extracted from the EMG data, a group 5 indicator, which is for example a mean signal peak-to-peak amplitude, or EMG-MSPA, wherein the EMG-MSPA, or Group 5 indicator, is indicative of the level of pain the subject consciously perceives.

The Group 5 indicator:

- EMG-MSPA

Is an indicator for the level of pain a subject consciously perceives, or in other words the LoP.

Similar as explained above with reference to the group 4 indicator, also for the group 5 indicator there can be made use of reference data to scale and/or index the measured data for determining the LoP. For example, a change of said EMG-MSPA compared with a reference value is further indicative of a change to the LoP of the subject. A reduction of said EMG-MSPA compared with a reference value is further indicative of a reduced LoP of the subject. It is clear that an increase compared to a reference value, is thus indicative of an increase of the LoP. It is clear that similar as described above with respect to group 4 indicator, also for a group 5 indicator, and/or any suitable combination of group indicators and/or other group indicators, there can be scaled and/or indexed a subsequently measured LoP with respect to the at least one predetermined reference LoP by means of said at least one correlation, which could be subject based or population based.

As already mentioned above, a level of pain or LoP of the subject may be determined from a Group 4 indicator and optionally, a Group 3 indicator and/or a Group 5 indicator.

In particular, the level of pain, LoP may be determined from:

- power(ta), such as for example one or more of the C-power (ta), the P-power (ta), and the F-power (ta) (Group 4 indicator), and optionally
- the MSPA, such as for example C-MSPA, and/or P-MSPA and/or F-MSPA (Group 3 indicator), and/or
- the EMG-MSPA (Group 5 indicator).

In particular, the level of pain LoP may be determined from:

- the power(ta), for example one or more of the C-power (ta), the P-power (ta), the F-power (ta) (Group 4 indicator),
- optionally the MSPA, for example the C-MSPA, and optionally P-MSPA and optionally F-MSPA (Group 3 indicator), and
- optionally the EMG-MSPA (Group 5 indicator).

In particular, the level of pain (LoP) may be determined from:

- the power(ta), for example the C-power (ta), and optionally the P-power (ta), and optionally the F-power (ta) (Group 4 indicator), and
- the MSPA, for example the C-MSPA, and optionally P-MSPA and optionally F-MSPA (Group 3 indicator), and
- optionally the EMG-MSPA (Group 5 indicator).

Described herein are one or more indexes, which are standardized indexes indicating the level of pain as perceived by the subject. Typically an index is standardized or normalized as compared to a reference. As mentioned above such a reference could for example be subject-based or population based.

Indexes described herein include a level of pain index (LoPI).

A level of pain index (LoPI) is a standardized index of the level of pain (LoP) indicating the level of pain as consciously perceived by the subject. Such an index also allows to determine how much the LoP has been modified, for example by a treatment session, in particular by a pharmacological, and/or non-pharmacological treatment session as compared to a reference state.

An index, for example one or more of LoPI, may have a scale with a first and a second limit. The first limit may represent a lowest level of pain, or alternatively a lowest level of pain associated with no pain and the second limit may represent the highest level of pain when compared to a reference state. The first limit may represent an initial state or a reference state of the subject and/or population. The first limit may represent a normal level of pain and the second limit may represent a highest level of pain. The first limit may represent a normal level of pain, and the second limit may represent a significantly increased level of pain. The first and second limits may be numerically represented e.g. 0 to 20, 20 to 0, 0 to 60, 60 to 0, 0 to 100, 100 to 0, 0 to 1, 1 to 0 respectively. The second limit may be higher than the first limit, or the first limit may be higher than the second limit. The choice of a higher number for the second limit may depend on the subject or user perspective. An anesthetist may have a preference that the first limit is higher (e.g. 1, 60, 100, 100%) than the second limit (e.g. 0, 0%).

A subject with an index, for example a LoPI which remains close to the first limit, for instance, can be safely operated on by surgery when the perception of the level of pain remains for example sufficiently low by means of suitable treatments causing an anesthetic effect.

The index may have a scale between the first and second limits; the scale may be linear, logarithmic, or other. The scale may be continuous, categorical, discrete, percentage, ratio.

The index, e.g. LoPI, may be indicative of a number of different levels of pain or different LoP, for the subject. The number of levels may be any, for instance 3 to 10, preferably 4 to 8 levels. There may be 4, 6, or 8 levels. The levels may divided be within the first and second limits. The division may be even (linear), logarithmic, or according to another scheme. The lowest level (e.g. 1st level) may correlate with or contain the first limit, the highest level (e.g. 4th level) may correlate with or contain the second limit. When an index is a value between 100 (first limit) and 0 (second limit) and the number of levels is 4, the 4 levels may be 100-76 (1st level), 75 to 51 (2nd level), 50 to 26 (3rd level), 25 to 0 (4th level). It is appreciated that the end points of the levels may allow a continuous numerical scale (not interrupted) into the next level. When the index is a value between 100 (first limit) and 0 (second limit) and the number of levels is 8, the 8 levels may be 100-87.5 (1st level), 87.5 to 75 (2nd level), 75 to 62.5 (3rd level), 62.5 to 50 (4th level), 50 to 37.5 (5th level), 37.5 to 25 (6th level), 25 to 12.5 (7th level), 12.5 to 0 to (8th level). The skilled person would be able to determine the extent of the scale (e.g. 100 to 0, or 60 to 0), the number of levels within the scale (e.g. 4, 6 or 8), the type of scale (e.g. linear, logarithmic) and the boundaries between the levels according to the situation.

The index may be a subject-based index in which state of each subject is compared to a reference state of the subject itself or of a group of subjects, or one or more particular populations. The index may be a population-based index in which the state of each subject is compared to a population-based reference. It is clear that alternatively, the index may also be scaled according to an absolute scale. It is clear that any suitable combination of relative, absolute, individual and/or population-based could be used to scale the index.

With respect to the subject-based index, a reference state e.g. a reference value for LoP, is for example measured for the subject before the treatment session. During or after the treatment session, based on response data comprising measured data, real-time values for one or more of LoP is measured for the subject. During the treatment session, based on a comparison between the reference value and the real-time LoP values, real-time values for one or more of LoPI is calculated.

With respect to the population-based index, a reference state e.g. a reference value for one or more of LoP is for example drawn from a database. The database for example comprises reference values that are based on population studies. The reference value may vary according to certain factors include age, gender, ethnicity, intervention characteristics hence, the reference value may be adapted according to the subject and/or the user. During, or after the treatment session, based on response data comprising measured data, real-time values for one or more of LoP is measured for the subject. During, or after the treatment session, based on a comparison between the database comprising the population based reference value and the real-time LoP values, real time values for one or more of LoPI is calculated.

It is clear that, alternatively, for example the LoP could be scaled according to a subject-based of population-based absolute scale, such that no further reference measurement is required, and that for example measurement of LoP to determine real-time values could be performed without the need for any treatment session. In other words, with a subject-based or population-based absolute scale it is possible to measure LoP in any suitable context.

The response data may be transformed into an index e.g. into a LoPI using an evaluation protocol. The evaluation protocol may comprise use of one or more of a mathematical (e.g. statistical) model, trained machine-leaning model, mathematical index, reference data. The evaluation protocol takes as input the response data, and outputs the index.

The evaluation protocol may include a step of extracting from the response data one or more of the Group 3 indicator, Group 4 indicator, Group 5 indicator.

The measured data may comprise one or more data components. A data component is attributed to a single measurement data, for example EEG data, EMG data, EDA data, ECG data, ... , a single observation data, for example movement, skin color, ... , or a single self-reported event data, for example a self-reported level of pain perception, ....

The evaluation protocol may weight the data components of the response data equally or differently. The weighting given to a data component depends, amongst other things, on relevance and precision of the component. For instance, the evaluation protocol may give EEG data may have a higher rating, reflecting its high relevance and precision. The evaluation protocol may use data components which have less relevance and precision, that are still indicative enough for the situation. In some situations, the response data may comprise a smaller number of data components that have high relevance and precision that are indicative enough for the situation. In some situations, the response data may comprise a larger number of data components that have lower relevance and precision that are indicative enough for the situation.

The evaluation protocol may be refined as more subjects are evaluated using the method. A method of refining the evaluation protocol may comprise:

- receiving response data of a subject, for example representing a subject's response to a treatment session,
- receiving independently measured data of LoP(I),
- using the response data and independently measured data to refine the evaluation protocol.

A method of refining the evaluation protocol may comprise:

- Receiving measured response data of a subject, for example representing a subject's response to a treatment session,
- Receiving self-reported and/or observational measure of LoP(I);
- Receiving independently measured data of LoP(I);
- Using the response data, the self-reported and/or observational data, and the independently measured data to refine the evaluation protocol.

The evaluation protocol may additionally or alternatively be refined using analytics. The data collected before, during and/or after one or more previous measurements, and/or one or more previous treatment sessions may contribute a refinement of the evaluation protocol.

As the evaluation protocol is created and/or refined, it may be apparent that one or more data components of the response data become redundant in determining and/or monitoring the level of Pain, or for example the modification of the Level of Pain by means of a pharmacological and/or non-pharmacological treatment, for example involving a pain treatment, a treatment involving the modification of the state of consciousness of a subject, etc.. According to a preferred embodiment at least one, preferably all of the measured data, observation data and response data are used to create and/or refine the evaluation protocol. According to a preferred embodiment only measured data is used in the method for measuring the LoP.

An embodiment of an evaluation protocol for determining a LoPI is set out below. Generally speaking, it may for example comprise 5 principal steps:

Step 1: receive and digitize response data comprising EEG data, for example during a treatment session. The EEG data could for example be provided from one or more of C-EEG, P-EEG and F-EEG electrodes, or any other suitable EEG electrodes and optionally from one or more EMG electrodes.

Step 2: generate:

Group 4 indicators power(ta), for example one or more of C-power (ta), P-power (ta), F-power (ta), from the digitized response data, and/or

Group 3 indicators MSPA, for example one or more of C-MSPA, P-MSPA, F-MSPA, and/or

Group 5 indicator EMG, for example EMG-MSPA.

Step 3: generate one or more indicator values, which means values for any of the group indicators, such as for example group 4 indicator, group 3 indicator, group 5 indicator, or any other suitable group indicators, from step 2), using for instance, integrative analysis.

Step 4: comparing the value of Step 3) with reference values, for example a population-based index or subject-based index such as mentioned above.

Step 5: obtain from Step 4) a LoPI index *e.g.* a number of a scale 0 to 20, or a word, or category.

In step 2, the digitized response data may undergo a pre-processing protocol including one or more of re-referencing to average reference, bandpass and/or notch filtering to exclude low and high frequency artifacts, signal segmentation into so called epochs (= sets of x seconds time windows), artifact rejection (ocular, muscular, ...), and baseline adjustment. For time domain analysis of EEG signals, there is typically peak detection to determine peak amplitude and latency (in form of a time series if multiple epochs), and mathematical transformations performed (e.g.: mean or change variable if multiple epochs). As already mentioned above in relation to the signals shown in FIG. 21, 22 and 23, in case there is only a single peak in the time window for measurement of the MSPA, the MSPA is equal to the peak-to-peak amplitude of the signal. In case there are multiple peaks in the signal in the time window for measurement of the MSPA, the MSPA will be calculated in function of the mean, average, weighted mean, etc. of the individual peak-to-peak amplitudes of the plurality of these peaks of the signals. The time window for measurement could for example be in the range of 0.01s to 10s, such as for example in the range of 0.1s to 5s.

For a frequency domain analysis of EEG signals: there is typically a time to frequency transform (e.g. Fourier transform), relevant frequency bands (*e.g.* theta-alpha (ta)) are extracted, amplitude/power and phase for each frequency band (and each epoch) are determined, and mathematical transformations performed (e.g.: mean if multiple epochs).

For each pair of EMG electrode (and each epoch) signals are analyzed in the time domain, peaks are detected, mean signal peak-to-peak amplitude (MSPA) and MSPA latency are determined, mathematical transformations performed (*e.g.*: mean if multiple epochs).

From this, one obtains at least one of the following features for at least one electrode: MSPA amplitude, MSPA power, MSPA latency, theta-alpha (ta) frequency band related amplitude/power, theta-alpha (ta) frequency band related phase, EMG MSPA, EMG MSPA latency. There may be a normalization step for instance to correct for the time of intervention to ensure that every subject contributes similarly.

In step 3, one or more group indicator values is generated using integrative analysis, for instance, using the equation:

$$LoP_{s,t} = \sum_{i=1}^{n} (\alpha_i \, f_i(F_{i,s,[t',t]}))$$

Where $\alpha_i$ is an optional parameter that evolves with the population size and characteristics, and may be determined using learning processes based on response data and/or independently measured data of the LoP, $f_i$ is a function determined using learning processes based on response data and/or independently measured data of the LoP, $F_{i,s[t',t]}$ is the value(s) of the feature obtained from point 2 from time t' to time t (t'<t) and for the subjects. The above-mentioned [t' - t] interval means that the value of LoP at time t may be computed based on features extracted from several $F_{i,s,t}$ values. As example, taking the mean and the standard deviation from the past x milli-seconds leads to a more sensitive and more robust determination of LoP. It is clear that the [t' - t] interval thus refers to the time-window mentioned above.

In step 4, there is a comparison of values with reference values

If the index is a subject-based index (each subject is its own control),

$$\Delta_r LoP_{s,t} = S \left( \beta \left( LoP_{s,p}^* - LoP_{s,[t',t]} \right) + K \right)$$

If the index is population-based (absolute) and that subject s is part of population p,

$$\Delta_a LoP_{s,t} = S \left( \beta \left( LoP_p^* - LoP_{s,[t',t]} \right) + K \right)$$

Where S is a scale-up parameter, $\beta$ is a parameter accounting for the population characteristics (age, sex, ethnicity, ...) determined using learning processes based on response data and/or independently measured data of the LoP, $LoP_{s,p}^*$ is the individual/subject-based reference state, $LoP_p^*$ is the absolute/population-based reference state using learning processes based on response data and/or independently measured data of the LoP, and K is a "translation" parameter allowing to adjust thresholds based on user preference.

In step 5 the LoPI index is generated. The value obtained in step 4 is compared with a predefined scale depending on subject/intervention characteristics determined following process described below in the section "refinement of the evaluation protocol", for example using trained machine-learning models and/or artificial intelligence, based on response data and/or independently measured data and/or reference data.

In LoPI, the index may be a population-based index, or in other words an absolute scale, in which a subject is compared with a population and/or any other suitable absolute reference. The first limit may indicate that the level of pain corresponds to no pain being perceived or being free from any pain as determined by the population. The second limit may indicate that the level of pain corresponds to perceiving the highest possible level of pain as determined by the population. Intermediate values represent intermediate levels of pain.

In LoPI, the index may be a subject-based index, or in other words a relative scale, in which each subject is compared with itself. The first limit may indicate that the level of pain, corresponds to no pain being perceived or being free from any pain as determined by the subject itself. The second limit may indicate that the level of pain corresponds to perceiving the highest possible level of pain as determined by the subject itself.

The method and system may determine an expected LoPI and/or ratio, for example for a point in time before, during and/or after the treatment session, for example based on the particular phase, acts of treatment already performed, etc. before, during and/or after the treatment session. For instance, if the treatment comprises a hypnotic session, an expected LoPI may be determined from the phase, for example one of phases (i) to (iv), as will be described in further detail below, of the hypnotic session, and for example from the position within such a phase. For instance, if the treatment comprises a pharmacological sedation session, an expected LoPI may be determined from the dose, titration, timing of the administration of the sedative. The expected LoPI may for example be determined from population data or subject data, namely from historical and/or precedence data.

Because an administered treatment does not always lead to the expected LoP a difference between the measured LoP and the expected LoP provides the user with guidance for corrective measures with respect to the treatment.

It is clear that still further alternative embodiments are possible, in which there is provided a computer-implemented method for determining and/or monitoring a level of pain of a subject, which comprises the method for measurement of a level of pain, according to one or more of the embodiments as described above, applied on a subject by receiving the measured data of a subject.

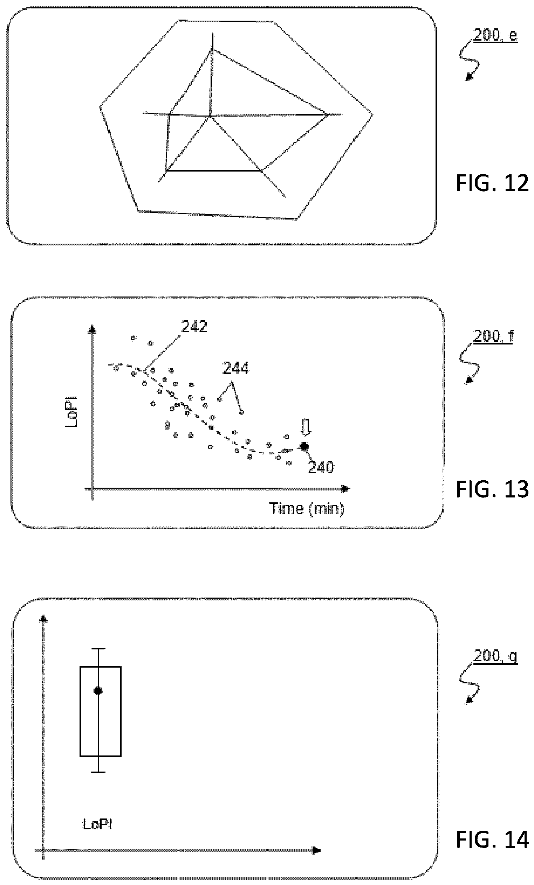

According to a further aspect, similar as described above, there is thus also provided a method for determining and/or monitoring a level of pain in a subject at at least two different points in time, for example before, during and/or after a treatment. According to such a method, there is measured a LoP at at least two different points in time, for example before, during and/or after the treatment. Based on a comparison of at least two LoP measured at at least two different points in time, there is then determined and/or monitored an evolution, for example a treatment-induced evolution of the LoP. However it is clear that such comparisons could be performed to determine any suitable evolution of the LoP during any suitable time period. Additionally and/or alternatively according to some embodiments the LoP could also be predicted. As for example described in further detail below with respect to Fig. 13 a suitable plurality of historical data points with respect to LoP could provide for a suitable trend analysis, for example based on a trend correlating to the a running average, mean, or any other suitable trend of the historical data points, thereby allowing prediction of future, expected or trending data points. It is clear that in this way a prediction of an evolution of the LoP can be accomplished. Predictive analysis of the historical data points, could be done by means of any suitable method, for example based on predetermined correlations between the evolution of LoP during different phases or time periods of a particular treatment, or by means of self-learning, self-optimizing algorithms, artificial intelligence algorithms, etc. which have been trained on historical data sets in order to detect repeating and/or predictable patterns and/or correlations, thereby allowing for an assessment of detectable patterns and/or correlations in the measured data, thereby allowing for a determination of current and predictive trends based on the measured data. It should be clear that alternative embodiments are possible of historical data points and/or any other suitable data resulting from LoP measurements and/or any other suitable measurement for enabling predictive analysis of current measurements. Such data on which the predictive analysis can be based can for example comprise historical data from individual subjects, from populations, predetermined treatment related patterns of an evolution of LoP, etc. According to still further embodiments, additionally and/or alternatively there may be aggregated a level of pain score or LoPS based on an aggregation of at least two LoP determined at at least two different points in time. Such aggregated parameters such as LoPS related to pain measurement could be useful in determining the impact of chronic pain, in which for example during a longer period, of for example several hours and/or days, the level of pain is continuously measured, and the aggregated LoPS is able to provide for an indicator of the impact of a constant, chronic level of pain as perceived by a subject as opposed to an instantaneous peak of pain. This is useful as even moderate or low levels of pain, when experienced chronically, may have a high impact on the quality of life of subjects, while for example short, infrequent bursts of pain, even when they are intense can be tolerated more easily. It is clear that such aggregated forms could also be useful for any parameters related to the level of pain as detailed in this description, and/or their scaled and/or indexed versions, such as for example LoP(I), etc. thereby allowing for similar scores of these parameters that correspond to the aggregation of at least two values of these parameters. According to particular embodiments the LoP(I)S could for example comprise any suitable embodiment of aggregation of the LoP(I) values over a predetermined time period, such as for example a sum, mean, average, etc. and the value could for example be presented in a GUI of a system similarly as described below, for example presenting the aggregated value, optionally in relation to the time period during which the LoP(I) was aggregated.

According to some treatments, such as for example pain treatments, in which for example a pharmacological and/or non-pharmacological treatment is used to reduce a particular level of pain the subject is experiencing for example by means of a sedative, it is clear that the goal of the treatment is to reduce the LoP, and that such a reduction in the LoP, for example during and/or after the treatment, corresponds to a desired treatment-induced evolution of the LoP.

However according to certain other treatments, for example in a context of an anesthetic treatment, for example prior to a chirurgical treatment, it could be desired to retain the LoP as experienced in the normal awake state, in which the subject does not undergo the chirurgical treatment, also during the chirurgical treatment. In this way, it is clear that the desired treatment induced evolution of the LoP is the prevention of an increase of the LoP, or the prevention of an increase of the LoP beyond an undesirable threshold. It is clear that according to such embodiments, the treatment-induced evolution of the LoP can for example be based on a comparison of the LoP measured before and during the treatment, or measured before and after the treatment, etc.. It is thus clear that, according to an embodiment in which the treatment is a pain reducing treatment, then the efficacy of such a pain treatment, such as for example a pain-reducing, pain-controlling, pain-preventing, or any other suitable pain treatment, can be determining and/or monitored based on the treatment-induced evolution of the LoP. In other words, when the LoP is sufficiently reduced, for example to below a desired threshold, or when the LoP does not undesirably increase, for example to above an undesired threshold. Preferably, this treatment-induced evolution of the LoP allows to optimizing and/or adapting a pain treatment, such as for example a pain-reducing, pain-controlling, pain-preventing, or any other suitable pain treatment.

It is further also clear that based on the measured LoP, there could be determining a treatment and/or determining an adjustment to a treatment and/or determining an intensity of a treatment. For example, a higher value for the LoP could lead to a higher dosage of a sedative. According to another example, if a particular pain treatment doesn't result in a decrease of the LoP, doesn't prevent an increase of the LoP, then an alternative treatment could be initiated. The desired level of titration of a pharmacological pain treatment, or the desired intensity of a non-pharmacological treatment, such as for example the depth of hypnosis, could for example be increased or decreased based on a desired LoP or treatment induced change thereto.

The method and system may provide an output to a graphical user interface (GUI). The system may comprise a GUI. As will be described in further detail below the system may comprise a graphical user interface, GUI, configured to indicate numerically and/or graphically one or more of:

- a historic, current and/or expected LoP or LoPI;
- the evolution of and/or ratio between two LoP or LoPI measured at two different points in time;
- optionally, one or more components of the measured data, preferably one or more EEG data and optionally EMG data.

It is clear that alternative embodiments of the GUI are possible in which further data is displayed, such as for example ECG data, heart rate data, respiratory data, ....

The output to the GUI may additionally or alternatively show one or more current data components (e.g. one or more measured data components, one or more observed data components, and/or one or more self-reported data components). Examples of current data components include those listed elsewhere herein, preferably one or more of EEG data, EMG data, ECG data, ....

The output to the GUI may comprise a graphical indicator wherein a position and/or color on the screen provide the user (e.g. doctor) an indication of the state of the subject and if necessary steps to be taken. For example, a green color may indicate a positive status in terms of patient experience of signal for the user. For example, a red color may indicate a negative status in terms of patient experience of signal for the user. For example, a left position may indicate a positive status in terms of patient experience of signal for the user. For example, a right position may indicate a negative status in terms of patient experience of signal for the user. For example, an upper position may indicate a positive status in terms of patient experience of signal for the user. For example, a lower position may indicate a negative status in terms of patient experience of signal for the user.

The output to the GUI may additionally or alternatively show one or more derived indexes. A derived index is an index derived from one or more data components and/or from another index. A derived index might be based on a ratio of LoPI. These derived indexes may be based on collected data as observed for the subject, optionally refined using analytics (i.e. historical/population data).

A graphical output may comprise a timeline and a marker on the timeline indicating the LoPI and/or a ratio thereof. The LoPI and/or a ratio thereof may be on scale with a first and second limit e.g. 0 to 1, 0 to 100.

The GUI may show the current LoPI on one or more scales between the first and second limits; the scale may be linear, logarithmic, or other. The scale may be continuous, categorical, discreet, percentage, ratio.

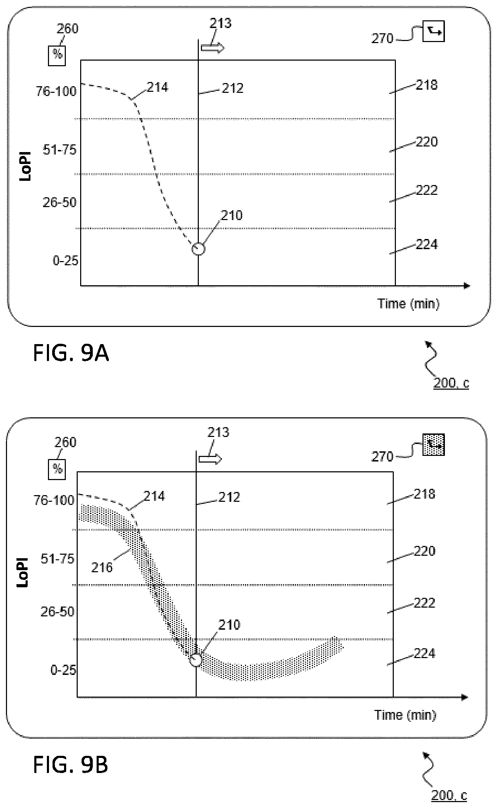

Examples of GUIs (200, a to g) are given in Figures 9 to 14.

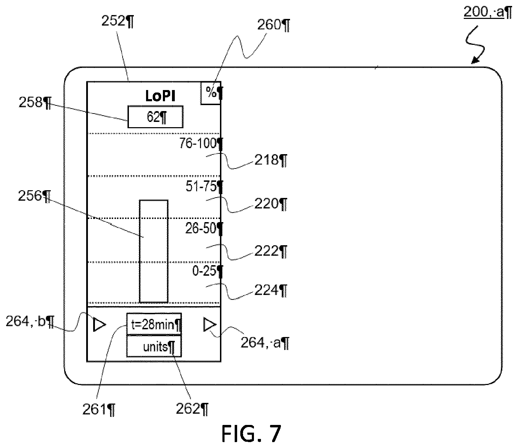

FIG. 7 shows a GUI (200, a) that is a graphical display of current status of LoPI as a bar chart (252). Reference is made to the LoPI bar chart (252). The LoPI (y-axis) bar chart displayed is divided into 4 zones: 0-25% (224), 26-50% (222), 51-75% (220), 76-100% (218); these may optionally be colored to indicate a level of safety (e.g. 76-100% - red, 51-75% - orange, 26-50% - light green, 0-25% - deep green. The 100-76% (218, red) zone may indicate that in his perception the subject is experiencing very high, undesirable amounts of pain, which might for example indicate the need to optionally administer analgesic. The 75-50% (220, orange) zone may indicate that the subject is perceiving a level of pain that is uncomfortable. The 50-26% (222, light green) zone may indicate that the subject is perceiving a light level of pain. The 0-25% (224, deep green) zone may indicate that the subject is not perceiving any pain or a level of pain that is not perceived as uncomfortable. It is appreciated that the units of percent and the extent of the scale of 0 to 100% is exemplary, and other units (e.g. unitless) and scales (e.g. 0 to 1, 0 to 40, 0 to 50, 0 to 60 etc., linear, logarithmic) are within the scope of the disclosure. The height of the bar (256) is indicative of the LoPI at the indicated time (261). A numerical display (258) also indicates the LoPI. Current units (percent) are indicated (260), that can be changed (e.g. to unitless and/or other units) with a units button (262). Forwards (264, a) and backwards (264, b) buttons allow the display to scroll back and forth between current LoPI and previous readings. The percentages on the LoPI bar, might scale similarly as for a pain scale as mentioned above, in which for example 0% corresponds to no pain and 100% corresponds to the most severe pain possible, as consciously perceived by the subject.

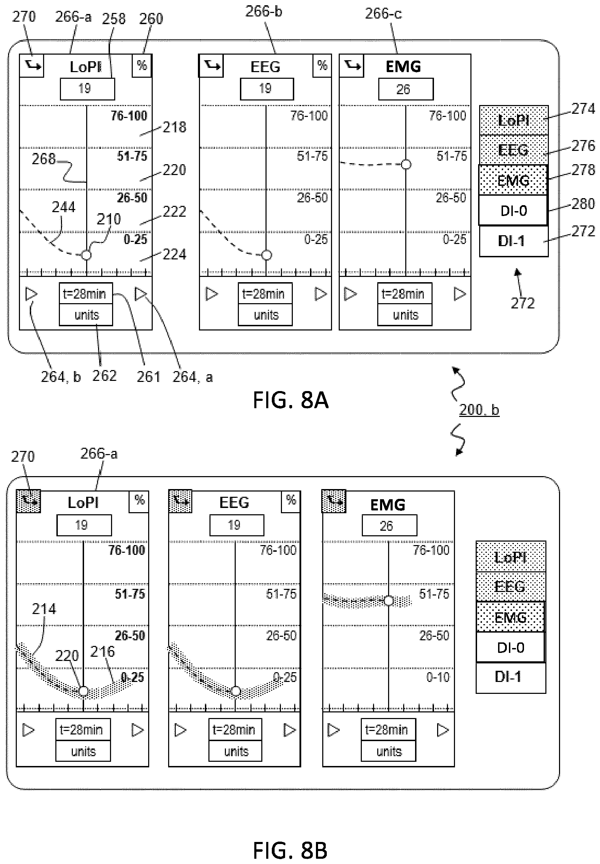

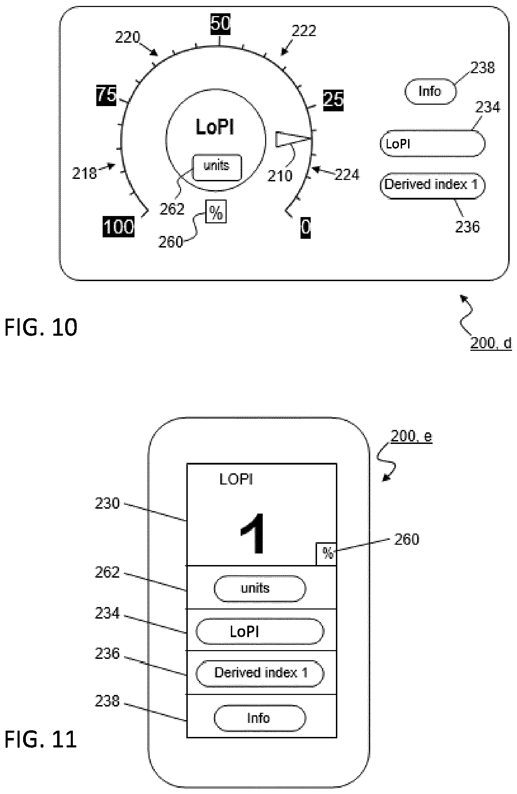

FIGs. 10A and 10B shows a GUI (200, b) that is graphical display of current status of LoPI, EEG and EMG as separate scroll charts (266-a 266-b, 266-c respectively). Reference is made to the LoPI scroll chart (266-a) in FIG. 8A; the other scroll charts contain equivalent features (not labelled). The LoPI scroll chart displayed is divided into 4 zones: 0-25% (224), 26-50% (222), 51-75% (220), 76-100% (218); these may optionally be colored to indicate a level of safety (e.g. 76-100% - red, 51-75% - orange, 26-50% - light green, 0-25% - deep green as in FIG. 7. The LoPI scroll chart (266-a) contains a stationary time axis (268) along which the LoPI reading (210) at the indicated time (261) slides (up and down). A numerical display (258) also indicates the LoPI. The time axis (268) remains static, while the background scrolls in the direction of the time (typically from right to left). Historical or trending datapoints (244) are indicated. Current units (percent) are indicated (260) that can be changed (e.g. to unitless and/or other units) with a 'units' button (262). Forwards (264, a) and backwards (264, b) buttons allow the display to scroll back and forth between current LoPI and previous readings. A panel of buttons (272) allows the user to choose from a selection of scroll charts for display: buttons for LoPI (274), EEG (276) and EMG (280) are selected (grey background), and the selected scroll charts are displayed (266-a, 266-b, 266-c respectively). Unselected are buttons for DI-0 (278, derived index-0) and DI-1 (derived index-1, 272)). In FIG. 8B, the same GUI as FIG. 8A are shown, and button (270) is unselected in FIG. 8A and is selected in FIG. 8B; button (270) toggles on or off a display of expected LoPI (216) for the subject that is superimposed on the scroll chart; current LoPI (210) and historical LoPI (214) can be visually compared with the expected LoPI (216).

FIG. 9A and FIG. 9B shows a GUI (200, c) that is graphical display of a progress of a treatment session over time (x-axis). Reference is made to the LoPI graph in FIG. 11A; the graph in FIG. 9B contains equivalent features (not labelled). The current LoPI (210) of the subject at the current point of time shown as a circle (210) along a time axis (212) that advances in the direction of arrow (213), for example as the treatment session progresses. The LoPI graph y-axis displayed is divided into 4 zones: 0-25% (224), 26-50% (222), 51-75% (220), 76-100% (218); these may optionally be colored to indicate a level of safety (e.g. 76-100% - red, 51-75% - orange, 26-50% - light green, 0-25% - deep green as in FIG. 7.

Historical or trending LoPI for the subject during the treatment session is shown as a dashed line (214). Current units (percent) are indicated (260); the box (260) may also serve as a button to cycle between different units (e.g. to unitless and/or other units). In FIG. 9B, the same graph as FIG. 9A is shown; button (270) is unselected in FIG. 9A and is selected in FIG. 9B. Button (270) toggles on or off a display of expected LoPI (216) for the subject that is superimposed on the graph; current LoPI (210) and historical LoPI (214) can be visually compared with the expected LoPI (216).

FIG. 10 shows a GUI (200, d) that contains a "speedometer" scale of the current LoPI (210) of the subject shown as a pointer. Current scale units (percent) are indicated (260), that can be changed (e.g. to unitless and/or other units) with a units button (262). The LoPI speedometer scale is divided into 4 zones: 0-25% (224), 26-50% (222), 51-75% (220), 76-100% (218); these may optionally be colored to indicate a level of safety (e.g. 76-100% - red, 51-75% - orange, 26-50% - light green, 0-25% - deep green as in FIG. 7. Buttons (234, 236, 238) are displayed from which the user can select one or more options to change the display of the GUI and for further information.

FIG. 11 shows a GUI (200, b) that contains a numerical display of the current LoPI (230) of the subject. Current units (percent) are indicated (260), that can be changed (e.g. to unitless and/or other units) with a units button (262). Buttons (234, 236, 238) are displayed from which the user can select one or more options to change the display of the GUI and for further information.

FIG. 12 shows a GUI (200, e) wherein different indexes are represented. External point of each axis represents the maximal scale value for this index. Chart gives an overview of the values of different indexes.

FIG. 13 shows a GUI (200, f) displaying current LoPI for the subject (240), for example during a therapeutic session, an anesthetics, analgesic treatment, etc. overtime. Historical or trending datapoints (244) are also shown, and an average of the historical or trending data points is shown as a dashed line (242).

FIG. 14 shows a GUI (200, g) displaying an average measure for the LoPI compared to the rest of the population (analytics), for example at the same point in time of the treatment session.

A system may be provided for determining and/or monitoring a Level of Pain of a subject, for example during a treatment session, the system comprising:

- optionally, a media renderer configured for presenting the treatment session to the subject;
- a monitoring apparatus configured to obtained measured data of the subject;
- a controller module configured for receiving measured data from the monitoring apparatus, and transforming the response data comprising the measured data into one or more of LoPI. The evaluation protocol may comprise use of one or more of a mathematical (e.g. statistical) model, trained machine-learning model, mathematical index, reference data.

It is thus clear that, according to an embodiment, there is provided a system configured to measure a level of pain according to the method for measuring the LoP as described above. Such a system comprises a monitoring apparatus configured to obtain measured data comprising electroencephalogram, EEG, data collected from one or more EEG electrodes. It further comprises a controller module configured for receiving the measured data from the monitoring apparatus; extracting from the EEG data, a group 4 indicator corresponding to a power, power (ta), associated with a theta-alpha frequency band, ta, within a theta-alpha frequency range; and determining, based on said group 4 indicator, a level of pain, LoP, which is a value indicative for the level of pain in the subject. Optionally, it is clear that according to similar embodiments as explained above, the LoP could be further determined by means of group 3 and/or group 5 indicators. Preferably the monitoring apparatus comprises, for obtaining measured data, one or more EEG electrodes configured for collection of electroencephalogram, EEG, data, and optionally an EMG capturing unit. It is clear, as will be explained in further detail below, that still further embodiments of the system are possible.

According to an embodiment the monitoring apparatus may be configured to capture response data of the subject, in particular measured data of the subject. The monitor apparatus comprises one or more units, each unit containing, depending on the type of unit one or more electrodes, sensors, cameras that capture measured data. The measured data component of each unit may or may not result from a processing of a signal captured by the one or more electrodes, sensors, cameras.

The monitoring apparatus may comprise an electroencephalogram (EEG) capturing unit. The response data i.e. measured data comprises outputted EEG data. EEG capturing unit may comprise at least two (e.g. 2, 3, 4, 5 or more), preferably a plurality of electrodes configured for acquiring electrical activity data from the subject brain. The electrodes may be configured for placement at predetermined positions across the subject's cephalic region; for instance to the frontal, parietal, and/or central region. An exemplary electrode configuration is 2 frontal electrodes, 2 lateral electrodes, 1 parietal electrode with respect to the head. However it is clear that alternative embodiments are possible comprising other electrodes at other scalp locations.

In a preferred configuration, the EEG capturing unit may comprise at least three electrodes:

- the frontal EEG electrode (F-EEG electrode) configured for attachment to or contact with the scalp anatomical region corresponding to the frontal lobe;
- the parietal EEG electrode (P-EEG electrode) configured for attachment to or contact the scalp anatomical region corresponding to the parietal lobe;
- the central EEG electrode (C-EEG electrode) configured for attachment to or contact the scalp anatomical region corresponding to the precentral and postcentral gyrus.

The EEG capturing unit may further comprise a ground or reference electrode. This is situated away from the F-, P- or C-EEG electrode to allow spatial reconstruction

The electrodes may be integrated into a wearable device e.g. wearable headset. The electrodes may be fixed to or detachable from the wearable device. The electrodes may be dry-contact electrodes. The electrodes may be held in place be gravity, or by a force (e.g. applied by a spring or elastic) or by adhesive. The electrodes may be reusable or one-time use. The electrodes may be integrated into a head-strap or fixing band. The electrodes may be integrated into a mask portion of a media renderer, as explained in further detail below. The electroencephalogram (EEG) capturing unit typically includes an amplifier for amplification of the detected signal. Signals are digitized for processing by a digital-to-analogue converter for processing by the processing unit. The sampling rate is typically at least 2.5 times higher than the highest frequency of interest. The digital-to-analogue converter may be separate or built into the processing unit.

According to an embodiment the monitoring apparatus may comprise an electromyography (EMG) capturing unit. The response data i.e. measured data comprises outputted EMG data. EMG capturing unit may comprise at least one (e.g. 1, 2, 3, 4, 5 or more), preferably a plurality of electrodes configured for acquiring electrical activity data from the subject muscle tissue. Where an EEG capturing unit is present, the EMG capturing unit may share at least one electrode with the EEG capturing unit. Similarly, where an EEG capturing unit is present, an EMG capturing unit may share at least one electrode with the EEG capturing unit. The electrodes may be configured for placement at predetermined positions across the subject's cranial and/or facial region; preferably to the frontal region. The electrodes may be integrated into a wearable device e.g. wearable headset. The electrodes may be fixed to or detachable from the wearable device. The electrodes may be dry-contact electrodes. The electrodes may be held in place be gravity, or by a force (e.g. applied by a spring or elastic) or by adhesive. The electrodes may be reusable or one-time use. The electrodes may be integrated into a head-strap or fixing band. The electrodes may be integrated into a mask portion of the media renderer. The EMG capturing unit typically includes an amplifier for amplification of the detected signal. Signals are digitized for processing by a digital-to-analogue converter for processing by the processing unit. The digital-to-analogue converter may be separate or built into the processing unit.

According to an embodiment the monitoring apparatus may comprise a respiratory data (RD) capturing unit. The response data i.e. measured data comprises outputted respiratory data. RD capturing unit may comprise at least one (e.g. 1, 2, 3, 4, 5 or more) sensor(s) configured for acquiring RD from the subject. The respiratory data may comprise one or more of respiration rate, respiration rate variability, inhalation pressure. The RD capturing unit may comprise one or more photoplethysmogram (PPG) sensors. A PPG sensor detects blood volume changes in vasculature below the skin; the sensor is typically optical. The respiration rate and respiration rate variability may be determined from the PPG sensor. A PPG sensor may be placed at temple area and/or forehead area. The respiration rate and respiration rate variability may be determined from the PPG sensor. One or more of the sensor(s) may be configured for placement at predetermined positions across the subject's cranial and/or facial region. The sensor(s) may be integrated into a wearable device e.g. wearable headset. The sensor(s) may be fixed to or detachable from the wearable device. According to an embodiment, the sensor(s) may be dry-contact sensor(s) and/or electrode(s). The sensor(s) may be held in place be gravity, or by a force (e.g. applied by a spring or elastic) or by adhesive. The sensor(s) may be reusable or one-time use. The sensor(s) may be integrated into a head-strap or fixing band. The sensor(s) may be integrated into a mask portion of the media renderer e.g. so as to place PPG sensors at the forehead and/or temple region. The RD capturing unit may comprise a wearable chest band containing a force transducer that measures the expansion and contraction of the chest; the respiration rate and respiration rate variability may be determined from the wearable chest band sensor. The RD capturing unit may comprise an airways air pressure detector that measures the air pressure (e.g. during inhalation or exhalation). The RD capturing unit typically includes an amplifier for amplification of the detected signal. Signals are digitized for processing by a digital-to-analogue converter for processing by the processing unit. The digital-to-analogue converter may be separate or built into the processing unit.

According to an embodiment, the monitoring apparatus may comprise a heart rate (HR) capturing unit. The response data i.e. measured data comprises outputted HR data. HR capturing unit may comprise at least one (e.g. 1, 2, 3, 4, 5 or more) sensor(s) and/or electrode(s) configured for acquiring heart data from the subject. The HR capturing unit may comprise one or more photoplethysmogram (PPG) sensors that detects blood volume changes vasculature below the skin; the sensor is typically optical. A PPG sensor may be placed at temple area and/or forehead area. The peaks of a captured PPG wave can be estimate the heart rate, heart rate variability as well as heartbeat interval, and blood pressure (two PPG sensors). The HR capturing unit may comprise one or more ECG electrodes, or any other suitable electrode configured for acquiring electrical activity data from the subject heart. Where an EEG or EMG capturing unit is present, the HR capturing unit may share an electrode with the EEG or EMG capturing unit. One or more of the sensor(s) and/or electrode(s) may be configured for placement at predetermined positions across the subject's cranial and/or facial region; they may be placed bilaterally. Where the sensors are PPG sensors, may be configured for placement at the forehead and/or temple region. The sensor(s) and/or electrode(s) may be integrated into a wearable device e.g. wearable headset. The sensor(s) and/or electrode(s) may be fixed to or detachable from the wearable device. The sensor(s) and/or electrode(s) may be dry-contact sensor(s) and/or electrode(s). The sensor(s) and/or electrode(s) may be held in place be gravity, or by a force (e.g. applied by a spring or elastic) or by adhesive. The sensor(s) and/or electrode(s) may be reusable or one-time use. The sensor(s) and/or electrode(s) may be integrated into a head-strap or fixing band. The sensor(s) and/or electrode(s) may be integrated into a mask portion of the media renderer e.g. so as to place PPG sensors at the forehead and/or temple region. The HR capturing unit typically includes an amplifier for amplification of the detected signal. Signals are digitized for processing by a digital-to-analogue converter for processing by the processing unit. The digital-to-analogue converter may be separate or built into the processing unit.

According to an embodiment the monitoring apparatus may comprise an electrodermal activity (EDA) capturing unit. The response data i.e. measured data comprises outputted EDA data. EDA capturing unit may comprise at least one (e.g. 1, 2, 3, 4, 5 or more), preferably a plurality of electrodes configured for acquiring electrical activity data from the subject skin tissue. Where an EEG or EMG capturing unit is present, the EDA capturing unit may share an electrode with the EEG or EMG capturing unit. The electrode(s) may be configured for placement at predetermined positions across the subject's cranial and/or facial region; preferably to the frontal region.

The electrode(s) may be integrated into a wearable device e.g. wearable headset. The electrode(s) may be fixed to or detachable from the wearable device. The electrode(s) may be held in place be gravity, or by a force (e.g. applied by a spring or elastic) or by adhesive. The electrode(s) may be reusable or one-time use. The electrode(s) may be integrated into a head-strap or fixing band. The electrode(s) may be integrated into a mask portion of the media renderer. The EDA capturing unit typically includes an amplifier for amplification of the detected signal. Signals are digitized for processing by a digital-to-analogue converter for processing by the processing unit. The digital-to-analogue converter may be separate or built into the processing unit.

According to an embodiment, the EDA capturing unit may be configured for measurement of galvanic skin response. The response data i.e. measured data comprises outputted GSR data. EDA capturing unit may comprise at least one (e.g. 1, 2, 3, 4, 5 or more), preferably a plurality of GSR electrodes configured for acquiring galvanic skin response. Where an EEG or EMG capturing unit is present, the EDA capturing unit may share a localized or reference electrode with the EEG or EMG capturing unit for acquiring galvanic skin response. The electrode(s) may be configured for placement at predetermined positions across the subject's cranial and/or facial region; preferably to the frontal or forehead region. The GSR electrode(s) may be integrated into a wearable device e.g. wearable headset. The GSR electrodes may be fixed to or detachable from the wearable device. The GSR electrodes may be dry-contact electrode(s). The GSR electrode(s) may be held in place be gravity, or by a force (e.g. applied by a spring or elastic) or by adhesive. The GSR electrode(s) may be reusable or one-time use. The GSR electrode(s) may be integrated into a head-strap or fixing band. The GSR electrode(s) may be integrated into a mask portion of the media renderer.

According to an embodiment, the monitoring apparatus may comprise an electrocardiogram (ECG) capturing unit. The response data i.e. measured data comprises outputted heart rate or ECG data. ECG capturing unit may comprise at least one (e.g. 1, 2, 3, 4, 5 or more), preferably a plurality of electrodes configured for acquiring electrical activity data from the subject heart. Where an EEG, EMG, or EDA capturing unit is present, the ECG capturing unit may share an electrode with the EEG, EMG, or EDA capturing unit. The electrodes being configured for placement at predetermined positions across the subject's chest, or any other suitable location where the heart ECG data can be captured. The electrodes may be integrated into a wearable device e.g. wearable headset. The electrodes may be fixed to or detachable from the wearable device. The electrodes may be held in place by gravity, or by a force (e.g. applied by a spring or elastic) or by adhesive. The electrodes may be reusable or one-time use. An electrode may be integrated into a head-strap or fixing band. An electrode may be integrated into a portion of the media renderer.

According to an embodiment, the monitoring apparatus may further comprise a physiological monitoring unit. The response data i.e. measured data comprises outputted physiological data. The physiological monitoring unit may comprise at least one (e.g. 1, 2, 3, 4 or more) sensor for acquiring subject's physiological data, in particular a component of the physiological data. The physiological data may relate to one or more of the following: pulse rate, heart rate, heart rate variation, blood pressure, respiration rate, respiration rate variability, inhalation pressure, exhalation pressure, brain oxygenation, blood O2 saturation (SpO2), regional and/or central blood O2 saturation, skin conductance, galvanic skin response, body temperature. The physiological data may relate to one or more of the following: respiration rate, respiration rate variability, inhalation pressure, heart rate.

According to an embodiment the one or more sensors may be integrated into a wearable device e.g. wearable headset. The one or more sensors may be fixed to or detachable from the wearable device. The one or more sensors may be held in place be gravity, or by a force (e.g. applied by a spring or elastic) or by adhesive. The one or more sensors may be reusable or one-time use. The one or more sensors may be integrated into a head-strap. The sensors may be integrated into a mask portion of a media renderer as explained in further detail below.

According to an embodiment, the monitoring apparatus may further comprise a body motion tracking unit. The response data i.e. measured data comprises outputted body motion tracking data. The body motion tracking unit may comprise at least one (e.g. 1, 2, 3, 4 or more) motion sensor for acquiring subject's body motion, in particular a component of the body motion tracking data. Examples of motion sensors include a 2- or 3-axis accelerometer, a gyroscope, one or more cameras, magnetic/induction transducers. The body motion includes movements of the head, limb (arms, legs, hands, knee, elbow). The body motion tracking unit may be a head movement tracking unit.

According to an embodiment the one or more motion sensors may be integrated into a wearable device e.g. wearable headset. The one or more motion sensors may be fixed to or detachable from the wearable device. The one or more motion sensors may be held in place be gravity, or by a force (e.g. applied by a spring or elastic) or by adhesive. The one or more motion sensors may be reusable or one-time use. The one or more motion sensors may be integrated into a head-strap. The motion sensors may be integrated into a mask portion of the media renderer.

According to an embodiment the monitoring apparatus may further comprise an eye-tracking unit. The response data i.e. measured data comprises outputted eye tracking data. The eye tracking unit may comprise at least one (e.g. 1, 2, 3, 4 or more) camera for monitoring movement of one or both eyes of the subject. The eye tracking unit may comprise at least one light source (e.g. visible, infrared) configured to illuminate the eye. Captures images may be analyzed using eye tracking software to determine subject's focus of attention, drowsiness, consciousness or other mental states. According to an embodiment the one or more cameras may be integrated into a wearable device e.g. wearable headset. The one or more cameras may be fixed to or detachable from the wearable device. The one or more cameras may be integrated into a mask portion of the media renderer.

According to an embodiment the monitoring apparatus may further comprise a facial expression capturing unit. The response data i.e. measured data comprises outputted facial expression data - e.g. emotions, nociception. The facial expression capturing unit may comprise at least one (e.g. 1, 2, 3, 4 or more) camera for monitoring facial expressions of the subject. The facial expression capturing unit may comprise at least one light source (e.g. visible, infrared) configured to illuminate the face. Captures images may be analyzed using facial expression recognition software to determine subject's facial expressions and responses, e.g. pain or (dis)comfort. The one or more cameras may be integrated into a wearable device e.g. wearable headset. The one or more cameras may be fixed to or detachable from the wearable device. The one or more cameras may be integrated into a portion of the media renderer.

According to an embodiment the monitoring apparatus may comprise an EEG capturing unit, an EMG capturing unit, an EDA capturing unit, an ECG capturing unit, a physiological monitoring unit, a head tracking unit, an eye tracking unit and a face capturing unit. The monitoring apparatus may comprise an EEG capturing unit, an EMG capturing unit, a HR capturing unit, and/or a respiratory data capturing unit.

According to a particular embodiment, in which the system is used during a non-pharmacological treatment session, the system may further comprise a media renderer that presents a treatment session to the subject. The treatment session may contain hypnosis and/or other evidence-based psychological and/or mind/body intervention. The media render may comprise a screen (e.g. LED, LCD, projector) on which moving images are displayed. The images immerse the attention of the subject and/or control the subject's experience and physiological response. The media render may comprise a sound transducer (e.g. earphone, headphone, speaker) to which audio is passed (e.g. music, dialogue, sound effects).

Preferably the media renderer is integrated as a wearable device, e.g. headset. The media rendered may be provided as a virtual reality headset, as an augmented reality headset, or mixed reality headset. Most preferably the media renderer is integrated into a wearable device that provides virtual/ augmented/ mixed/ etc. reality; which typically includes a display or projector, stereo sound (mono, stereo, multidimensional), and head motion tracking sensors (e.g. gyroscopes, accelerometers, structured light systems, etc.). Examples of suitable headsets are those supplied by Oculus (e.g. Oculus Rift, Oculus Go), Pico (e.g. G2 4K, G2 Pro), LG electronics (e.g. LG 360 VR), HTC (e.g. HTC Vive), Samsung (e.g. Samsung Gear VR), Google (e.g. Google Cardboard), Microsoft (Hololens), and other off-the-shelf or customized designs. The media renderer may be expanded by also rendering somatosensory content, such as vibrations (e.g. vibration modules equipped in a seat), and/or olfactory content (e.g. releasing one or more fragrances into the nasal cavity). Optionally, the media renderer may be equipped with a computing unit for executing or playing data, or it may receive data from an external media server (i.e. streaming).

As mentioned previously, the media renderer may be integrated into a wearable device, e.g. headset. The monitoring apparatus may be integrated into the wearable device. One or more electrodes and/or one or more sensors, and/or one or more cameras of the monitoring apparatus may be integrated into the wearable device

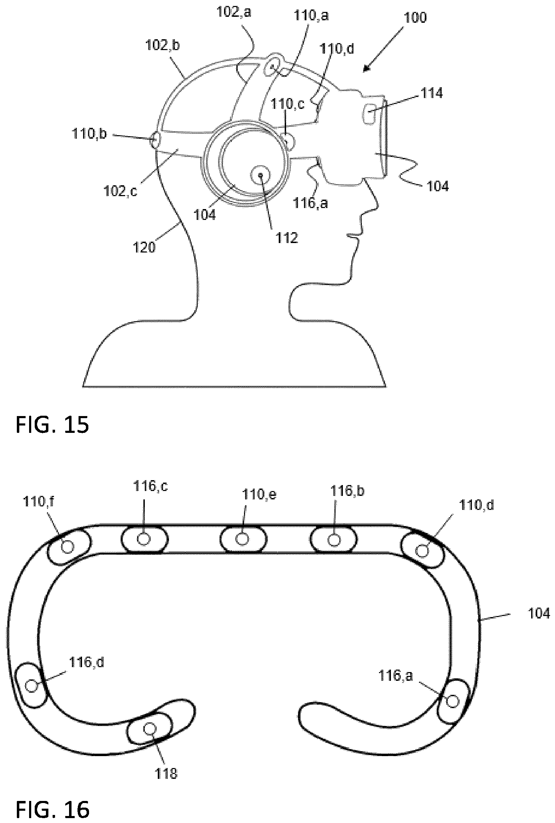

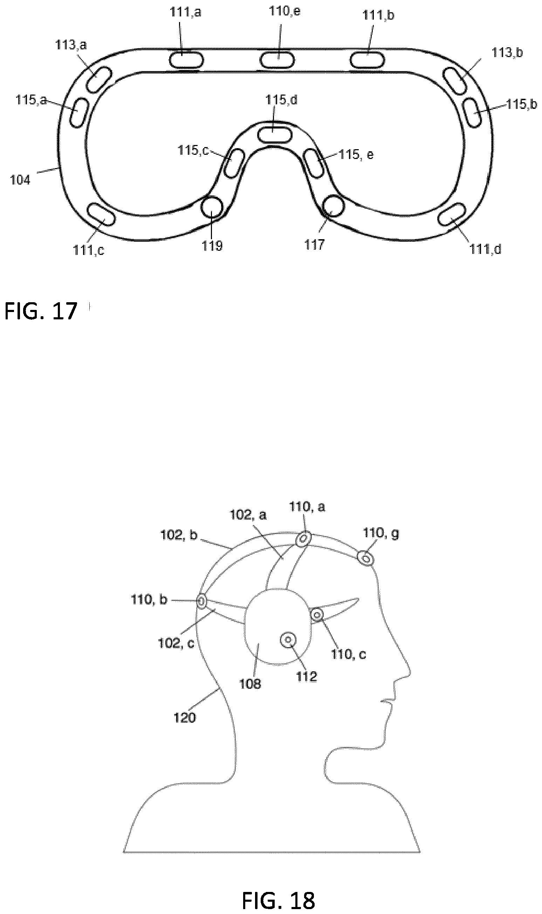

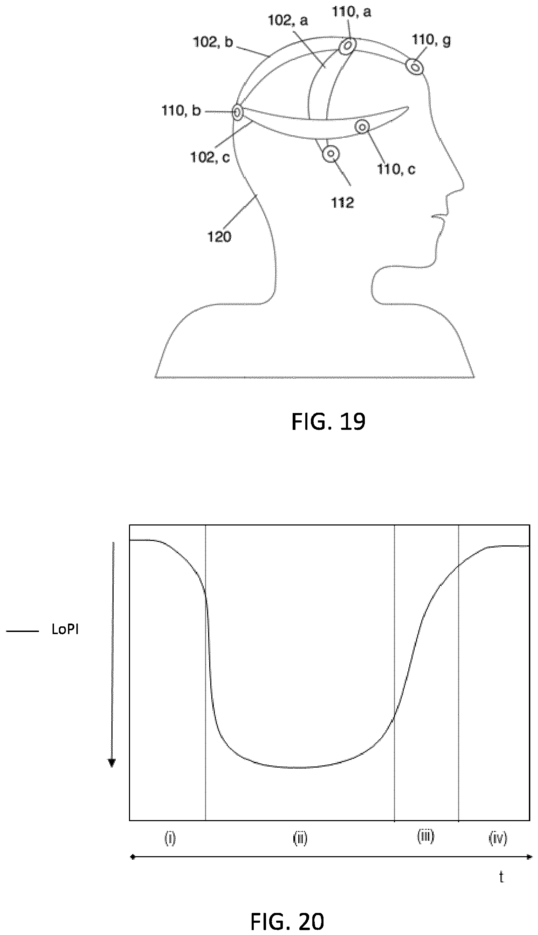

An exemplary embodiment of a wearable device is given in FIG. 15, Fig. 16 and Fig. 17. FIG. 15 depicts an embodiment of a wearable device (100) comprising a media rendered integrated into a virtual reality viewer (104). The wearable device (100) further comprises a plurality of elasticated straps (102,a,b,c) that hold an eye mask (104) supporting a virtual reality viewer and headphones (104) in position on the head (120) of the subject. It is however clear that alternative embodiments are possible of the wearable device (100), which for example do not comprise the eye mask such as for example shown in Fig. 18 and/or the headphones such as for example shown in Fig. 19, but only the electrodes and sensors. The elasticated straps (102,a,b,c) also hold a plurality of electrodes and sensors in position on the head (120) of the subject. Depicted are electrodes (110, a (central (C) EEG electrode), b (parietal (P) EEG electrode), c (temporal), d (frontal (F) EEG electrode)) for measurement of EEG data; sensor (112) for measurement of heart rate, heart rate variation, SPO2; and camera (114) for capture of eye movement and facial expression. FIG. 16 is a view of a face-contacting edge of the embodiment of a mask disposed with electrodes (116, a, b, c, d) for measurement of EEG data; electrode (118) for measurement of skin conductance. FIG. 17 is a view of a face-contacting edge of an embodiment of the mask (104) disposed with

-    a frontal (F) EEG electrode (110e) for measurement of EEG data;
-    EMG electrode (111a to 111d) for measurement of EMG data;
-    PPG sensors (113a, 113b) for measurement of respiratory data, heart rate data, blood pressure, spO2;
-    EOG electrodes (115a to 115e) for measurement of EOG data;
-    GSR electrode (117) for measurement of GSR data;
-    ground electrode (119) that can act a reference/ground electrode in combination with one or more of the EEG (110e), EMG (111a to 111d), EOG (115a to 115e), GSR (117) electrodes.

However it is clear that the system and/or wearable device may make use of any other suitable configuration and/or arrangement of EEG electrodes, such as for example devices comprising one or more electrodes, for example two, three, four or more electrodes arranged at one or more scalp locations referred to in the international 10-20 system, or any other suitable device comprising one or more EEG electrodes.

According to an embodiment, the controller module may be configured for receiving measured data from the monitoring apparatus, and transforming the response data comprising the measured data into one or more of LoPI. The evaluation protocol may comprise use of one or more of a mathematical (e.g. statistical) model, trained machine-leaning model, mathematical index, reference data.

According to an embodiment, the controller module may be configured for receiving measured data from the monitoring apparatus, and transforming the response data comprising the measured data into the LoPI, which is representing the level of pain as consciously perceived by the subject, using an evaluation protocol as described above. The evaluation protocol may for example comprise use of one or more of a mathematical (e.g. statistical) model, trained machine-leaning model, mathematical index, reference data.

According to an embodiment, the controller module typically comprises a circuit (e.g. microprocessor) configured to perform processing steps and memory. The controller module may or may not be integrated into a wearable device.

The method may be a computer implemented method.

There is further provided is a computing device or system configured for performing the method as described herein.

There is provided a computer program or computer program product having instructions which when executed by a computing device or system causing the computing device or system to perform the method as described herein.

There is provided a computer readable medium having stored thereon instructions which when executed by a computing device or system cause the computing device or system to perform the method as described herein.

There is provided a data stream which is representative of a computer program or computer program product having instructions which when executed by a computing device or system cause the computing device or system to perform the method as described herein.

The method and system described herein enable the therapist to quantify the patients level of Pain as consciously perceived by the subject, ... and/or enable optimal and potentially individual titration of initial dosages of analgesics, anesthetics, anxiolytics; ... hypnotics, and/or optimize pharmacological and/or non-pharmacological sedation (VR therapy titration); and/or provide safer progress by Quantifying, Visualizing, Trending and potentially Predicting the patient's physiological reaction / consciously perceived level of pain during clinical, medical, surgical or therapeutic interventions; and/or increase subject safety; and/or increase subject amnesia of medical intervention.

Also provided is use of a computer-implement method for measuring LoP as described herein before, during and after a treatment providing pharmacologically and/or non-pharmacologically induced sedation of a subject. Use of the computer-implemented method for measuring LoP also functions reliably before, during and after a treatment involving sedation, which may for example be provided for a surgical intervention and/or for example replacing and/or supplementing a pharmacological anesthetic with a non-pharmacological anesthetic.

It is to be understood that this invention is not limited to particular systems and methods or combinations described, since such systems and methods and combinations may, of course, vary. It is also to be understood that the terminology used herein is not intended to be limiting, since the scope of the present invention is determined by the appended claims.

As used herein, the singular forms "a", "an", and "the" include both singular and plural referents unless the context clearly dictates otherwise.

The terms "comprising", "comprises" and "comprised of" as used herein are synonymous with "including", "includes" or "containing", "contains", and are inclusive or open-ended and do not exclude additional, non-recited members, elements or method steps. It will be appreciated that the terms "comprising", "comprises" and "comprised of" as used herein comprise the terms "consisting of", "consists" and "consists of".

The recitation of numerical ranges by endpoints includes all numbers and fractions subsumed within the respective ranges, as well as the recited endpoints.

The term "about" or "approximately" as used herein when referring to a measurable value such as a parameter, an amount, a temporal duration, and the like, is meant to encompass variations of +/-10% or less, preferably +/-5% or less, more preferably +/-1% or less, and still more preferably +/-0.1% or less of and from the specified value, insofar such variations are appropriate to perform in the disclosed invention. It is to be understood that the value to which the modifier "about" or "approximately" refers is itself also specifically, and preferably, disclosed.

Whereas the terms "one or more" or "at least one", such as one or more or at least one member(s) of a group of members, is clear *per se*, by means of further exemplification, the term encompasses *inter alia* a reference to any one of said members, or to any two or more of said members, such as, *e.g.*, any $\geq 3$, $\geq 4$, $\geq 5$, $\geq 6$ or $\geq 7$ etc. of said members, and up to all said members.

All references cited in the present specification are hereby incorporated by reference in their entirety. In particular, the teachings of all references herein specifically referred to are incorporated by reference.

Unless otherwise defined, all terms used in disclosing the invention, including technical and scientific terms, have the meaning as commonly understood by one of ordinary skill in the art to which this invention belongs. By means of further guidance, term definitions are included to better appreciate the teaching of the present invention.

In this description, different aspects of the invention are defined in more detail. Each aspect so defined may be combined with any other aspect or aspects unless clearly indicated to the contrary. In particular, any feature indicated as being preferred or advantageous may be combined with any other feature or features indicated as being preferred or advantageous.

Reference throughout this specification to "one embodiment" or "an embodiment" means that a particular feature, structure or characteristic described in connection with the embodiment is included in at least one embodiment of the present invention. Thus, appearances of the phrases "in one embodiment" or "in an embodiment" in various places throughout this specification are not necessarily all referring to the same embodiment, but may. Furthermore, the particular features, structures or characteristics may be combined in any suitable manner, as would be apparent to a person skilled in the art from this disclosure, in one or more embodiments. Furthermore, while some embodiments described herein include some but not other features included in other embodiments, combinations of features of different embodiments are meant to be within the scope of the invention, and form different embodiments, as would be understood by those in the art. For example, in the appended claims, any of the claimed embodiments can be used in any combination.

In the present description of the invention, reference is made to the accompanying drawings that form a part hereof, and in which are shown by way of illustration only of specific embodiments in which the invention may be practiced. Parenthesized or emboldened reference numerals affixed to respective elements merely exemplify the elements by way of example, with which it is not intended to limit the respective elements. It is to be understood that other embodiments may be utilized and structural or logical changes may be made without departing from the scope of the present invention. The following detailed description, therefore, is not to be taken in a limiting sense, and the scope of the present invention is defined by the appended claims.

The inventors have found that for the first time a strong association between response data in particular measured data collected from at least one EEG electrode, such as for example at least one of a frontal-EEG electrode, a parietal -EEG electrode and central-EEG electrode and the LoP of the subject, for example during a pharmacological and/or non-pharmacological treatment session to modify the perception of the pain by the subject, such as for example by means of modifying the state of consciousness of the subject. For the first time, an objective measure of a level of pain of the subject can be determined in real time. This is applicable in many situations, for instance, when a subject is undergoing a surgically invasive procedure under pharmacological and/or non- pharmacological sedation.

State of consciousness refers to a measurement of a subject's wakefulness and/or self-awareness as well as environmental awareness determining the subject's presence sensation, arousability (emotional response) and (physical) responsiveness to an internal or external stimulus or stimuli, characterized by the physiological activity and/or by a neurological signature.

In specific state of the subject in which he perceives less or no pain, such as for example a specific modified state of consciousness, for example where the level of consciousness is different or modified with respect to a normal awake state, the subject for example perceives less or no pain and/or is optionally sedated. In such a specific state of the subject, the subject's cognitive functions may be absorbed in a specific task, and brain processes which usually happen together are separated. The creation such a specific state with reduced or no pain perception of the subject, which is for example a dissociative state allows a reduced perception of peripheral stimuli and a subject's modified perception of the self and the self in the environment. Subject's physiology is usually modified (less involuntary movement i.e. swallowing, limb movement, eye blinking, higher stability of vital signs, larger respiratory patterns and improved oxygenation rates...). In such a specific state with a reduced or no pain perception, such as for example a modified state of consciousness, a subject might experience partial or total catalepsy as well as disconnect from the environment (i.e. not hear/ respond to verbal command). Senses may be under/overactivated according to requirements. For example, a subject could be induced into such a specific state with a reduced or no pain perception, such as for example a modified state of consciousness, in which the dissociative state suppresses the conscious perception of pain sufficiently so that it is possible to safely undergo a surgical intervention, for instance.

According to an embodiment a treatment session is applied to the subject to modify the perception of pain of the subject, for example by modifying the state of consciousness of the subject. The treatment session may include one or more of hypnosis and/or other evidence-based psychological and/or mind/body intervention (i.e. non-pharmacological treatment), administration of active pharmacological ingredient (i.e. pharmacological treatment), other treatments (e.g. acupuncture, mechanical treatment, other non-pharmacological treatment). Preferably it comprises hypnosis.

In particular a non-pharmacological treatment includes mainly hypnosis but also other treatments which are used to potentialize the hypnosis session and/or decrease pain perception and/or potentialize the therapeutic impact. It is clear that alternative non-pharmacological treatment sessions are possible. Examples of pharmacological treatment sessions may comprise administration of an analgesic and/or an anesthetic. Examples of analgesics and/or anesthetics include:

- An inhalation agent such as Desflurane, Enflurane, Halothane, Isoflurane, Methoxyflurane, Nitrous oxide, Sevoflurane (inhaled)
- An intravenous agent such as Barbiturates, Amobarbital, Methohexital, Thiamylal, Thiopental, Benzodiazepines, Diazepam, Lorazepam, Midazolam, Etomidate, Ketamine, Propofol

As used herein the term "subject" refers to the beneficiary of the therapeutic session. The "user" refers to a person or persons operating the method or system. The user may be a care provider such as a physician, or medical assistant, or non-medical assistant (*e.g.* friend, relative, helper) of the subject. In some circumstances, the user may be the subject, for instance, where a treatment is self-administered at home.

Hypnosis is one way of inducing an altered state of consciousness in a subject. The hypnosis therapy may be medical (clinical) hypnosis therapy, or home hypnosis therapy, or hypnosis therapy provided in any care or wellness environment. The hypnosis therapy may be extramural hypnosis therapy that is not provided in a care institution (i.e. not provided in a clinic, hospital, care center). While immersed in the altered state of consciousness, the subject's self-perception and the peripheral awareness are affected, changing the subject's experience of his/her sensation, perception, and thoughts, and making the subject prone to follow suggestions. Subject is absorbed and dissociated from reality.

An embodiment of a hypnotic treatment session, such as for example an automated hypnotic treatment session 548 of experiment 500 as described above, typically comprises 4 sequential phases as exemplified in FIG. 20, (i) an induction phase, (ii) a deepening phase, (iii) a transition phase, and/or (iv) a re-alerting phase. While immersed in the treatment sessions the subject's self-perception and the peripheral awareness are affected, the immersion depth increasing during the (i) phase and reaching a maximum during the (ii) phase. The subject becomes prone to suggestive control during the (ii) phase, during which time the subject may be sedated, induced to relax, etc. Afterwards the subject is returned to a normal state of a consciousness by passing the (iii) and (iv) phases.

The phases are described in detail below, namely:

(i) The induction phase, wherein the subject is prepared to be immersed into the altered state. In this phase the subject is typically provided with a feeling of comfort, safety and relaxation to the subject.

(ii) The deepening phase, wherein the subject is placed in an altered state of consciousness. This state is typically characterized by full dissociation of reality and lack of or reduced movement unless expressly suggested in the therapy.

(iii) The transition phase, wherein the subject is exposed to suggestive information, which may aid in remembering or forgetting specific event of the therapy and/or to addressing one or more subject specific issues (also called post-hypnotic suggestions).

(iv) Re-alerting phase, wherein the subject is returned to a normal state of consciousness. In this phase the subject typically returns his/her senses and the dissociative state ends.

The hypnotic treatment session may be delivered by a hypnotist, more preferably it is presented using a media renderer such as a virtual reality head set for a fully immersive effect as described below in more detail. A stored hypnotic treatment session may be played through the media renderer, the content of the treatment session may be specific to the treatment goals.

While in the altered state of consciousness induced by hypnosis or other treatments, the subject can better manage pain as well as other symptoms/ issues. The subject can undergo medical interventions (*e.g.* invasive procedures) in the absence or with a lower dose of a pharmacological (anesthetic/analgesic/ anxiolytic) agent.

**Claims**

1. A computer-implemented method for measurement of a level of pain, the method comprising the steps of:

- receiving measured data comprising electroencephalogram, EEG, data;
- extracting from the EEG data, a group 4 indicator corresponding to:

- a power, power (ta), associated with a theta-alpha frequency band, ta, within a theta-alpha frequency range; and

- determining, based on said group 4 indicator, a level of pain, LoP, which is a value indicative for the level of pain in the subject.

2. The method according to claim 1, wherein the theta-alpha frequency band, ta, comprises one or more of the following:

- a frequency band encompassing both theta and alpha brain waves;
- a frequency band extending from 6Hz up to and including 12Hz; and/or

- a bandwidth of at least 2Hz and a frequency band comprising at least fast theta waves extending from 6Hz up to and including 8Hz, and/or

wherein the theta-alpha frequency range:

- comprises a frequency range encompassing both theta and alpha brain waves; and/or
- extends from 4 Hz up to and including 12 Hz.

3. The method according to claim 1 or 2, comprising the step of:

- extracting from the EEG data, a group 3 indicator corresponding to:

  - a mean signal peak-to-peak amplitude, MSPA; and

- determining the LoP based on the group 4 indicator and at least the group 3 indicator.

4. The method according to any of the preceding claims, comprising the step of:

- receiving measured data comprising electromyography, EMG, data;
- extracting from the EMG data, a group 5 indicator corresponding to:

  - a mean signal peak-to-peak amplitude, EMG-MSPA; and

- determining the LoP based on the group 4 indicator and at least the group 5 indicator.

5. The method according to claim 4, when dependent on claim 3, comprising the steps of:

- determining the LoP based on the group 4 indicator, the group 3 indicator, and the group 5 indicator.

6. The method according to any of the preceding claims, wherein the method comprises the step of:

- receiving the measured data of the subject comprising the electroencephalogram, EEG, data collected from one or more of the following EEG electrodes:

  - a frontal (F) EEG electrode configured for collection of F-EEG electrode data from the scalp anatomical region corresponding to a frontal lobe of the subject,
  - a parietal (P) EEG electrode configured for collection of P-EEG electrode data from the scalp anatomical region corresponding to a parietal lobe of the subject,
  - a central (C) EEG electrode configured for collection of C-EEG electrode data from the scalp anatomical region corresponding to a precentral and postcentral gyrus of the subject.

7. The method according to any of the claims 1 to 6, wherein the method comprises the steps of:

- receiving reference data comprising measured data of a subject and/or a population correlated to at least one predetermined reference LoP;
- determining at least one correlation of at least one predetermined reference LoP and at least one group indicator extracted from the reference data; and
- scaling and/or indexing a subsequently measured LoP with respect to the at least one predetermined reference LoP by means of said at least one correlation, and optionally determining a level of pain index or LoPI therefrom.

8. A computer-implemented method for determining and/or monitoring a level of pain of a subject, comprising the method for measurement of a level of pain according to any of the preceding claims applied on a subject by receiving the measured data of a subject.

9. A computer-implemented method for determining and/or monitoring and/or predicting and/or aggregating a level of pain in a subject before, during and/or after a treatment, wherein the method comprises the steps of:

- measurement of the LoP according to any of the claims 1 to 8, at at least two different points in time, preferably before, during and/or after the treatment;
- determining and/or monitoring and/or predicting and/or aggregating an evolution, preferably a treatment-induced evolution of the LoP, based on a comparison of at least two LoP measured at at least two different points in time; and/or
- aggregating a level of pain score or LoPS based on an aggregation of at least two LoP measured at at least two different points in time.

10. The method according to claim 9, wherein the method comprises the step of:

- determining the treatment-induced evolution of the LoP, based on a comparison of the LoP measured during and/or after the treatment with the LoP measured before the treatment.

11. The method according to claim 9 or 10, wherein the treatment is a pain reducing treatment and the method comprises the step of:

- determining and/or monitoring the efficacy of a pain treatment based on the treatment-induced evolution of the LoP.

12. The method according to any of the claims 9 to 11, wherein the treatment is a pain treatment and the method comprises the step of:

- optimizing and/or adapting a pain treatment based on the treatment-induced evolution of the LoP.

13. A computer-implemented method for determining a treatment and/or determining an adjustment to a treatment and/or determining an intensity of a treatment, wherein the method comprises the steps of:

- measuring a LoP according to any of the claims 1 to 7;
- determining a treatment and/or determining an adjustment to a treatment and/or determining an intensity of a treatment based on the measured LoP.

14. A system configured to measure a level of pain according to the method according to any of the preceding claims, the system comprising:

- a monitoring apparatus configured to obtain measured data comprising electroencephalogram, EEG, data collected from one or more EEG electrodes;
- a controller module configured for:

  - receiving the measured data from the monitoring apparatus;
  - extracting from the EEG data, a group 4 indicator corresponding to:

    - a power, power (ta), associated with a theta-alpha frequency band, ta, within a theta-alpha frequency range; and

  - determining, based on said group 4 indicator, a level of pain, LoP, which is a value indicative for the level of pain in the subject.

15. A system according to claim 14, wherein the controller module is further configured for:

- extracting from the EEG data, a group 3 indicator corresponding to:

  - a mean signal peak-to-peak amplitude, MSPA; and

- determining the LoP based on the group 4 indicator and at least the group 3 indicator.

16. A system according to claim 14 or 15, wherein the monitoring apparatus comprises, for obtaining measured data, one or more of:

- one or more EEG electrodes configured for collection of electroencephalogram, EEG, data;

- one or more frontal (F) EEG electrodes configured for collection of F-EEG electrode data from the scalp anatomical region corresponding to a frontal lobe of the subject,
- one or more parietal (P) EEG electrodes configured for collection of P-EEG electrode data from the scalp anatomical region corresponding to a parietal lobe of the subject,
- one or more central (C) EEG electrodes configured for collection of C-EEG electrode data from the scalp anatomical region corresponding to a precentral and postcentral gyrus of the subject;
- optionally an EMG capturing unit;
- optionally a heart rate capturing unit;
- optionally a respiratory data capturing unit.

17. The system according to any of the claims 14 to 16, further comprising a graphical user interface, GUI, configured to indicate numerically and/or graphically one or more of:

- at least two LoP respectively measured at different points in time, preferably

a historic, current and/or expected LoP.

- the evolution of and/or ratio between and/or aggregation of at least two LoP measured at different points in time;
- optionally, one or more components of the measured data, preferably one or more EEG data and optionally EMG data.

FIG. 1

FIG. 2

FIG. 3

FIG. 4

FIG. 5

FIG. 6

252¶

260¶

200, a¶

258¶

**LoPI**

62¶

%¶

76-100¶

218¶

51-75¶

220¶

256¶

26-50¶

222¶

0-25¶

224¶

264, b¶

t=28min¶

units¶

264, a¶

261¶

262¶

# FIG. 7

FIG. 8A

FIG. 8B

FIG. 9A

FIG. 9B

FIG. 10

FIG. 11

200, e

FIG. 12

200, f

FIG. 13

200, g

FIG. 14

FIG. 15

FIG. 16

FIG. 17

FIG. 18

FIG. 19

FIG. 20

Time domain response at central EEG electrode

FIG. 21

Time domain response at Parietal EEG electrode

FIG. 22

Time domain response at Frontal EEG electrode

FIG. 23

Europäisches
Patentamt

European
Patent Office

Office européen
des brevets

# EUROPEAN SEARCH REPORT

Application Number

EP 20 17 8463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2017/311882 A1 (SAAB CARL [US]) 2 November 2017 (2017-11-02) * paragraphs [0005], [0010], [0018], [0036], [0053], [0054], [0057], [0109] * | 1-17 | INV. A61B5/0476 A61B5/00 |
| X | US 2014/066739 A1 (HE BIN [US] ET AL) 6 March 2014 (2014-03-06) * paragraphs [0010], [0023], [0025], [0031] - [0033], [0060], [0065]; figure 2 * | 1-17 | |
| X | US 2017/135631 A1 (ZUCKERMAN-STARK GALIT [IL] ET AL) 18 May 2017 (2017-05-18) * paragraphs [0017], [0054], [0171], [0182], [0191]; figure 1; table 1 * | 1-17 | |
| X | US 2016/278697 A1 (JOHN ERWIN R [US] ET AL) 29 September 2016 (2016-09-29) * paragraphs [0011], [0013] - [0016]; figure 1 * | 1-17 | |
| X | US 2018/228421 A1 (SAAB CARL [US]) 16 August 2018 (2018-08-16) * paragraphs [0004], [0030], [0079]; claim 9; figure 1D * | 14-17 | TECHNICAL FIELDS SEARCHED (IPC) A61B |
| X | NIR RONY-REUVEN ET AL: "Tonic pain and continuous EEG: Prediction of subjective pain perception by alpha-1 power during stimulation and at rest", CLINICAL NEUROPHYSIOLOGY, vol. 123, no. 3, 1 November 2011 (2011-11-01), pages 605-612, XP028887575, ISSN: 1388-2457, DOI: 10.1016/J.CLINPH.2011.08.006 * the whole document * | 1-17 | |

-/--

The present search report has been drawn up for all claims

| Place of search | Date of completion of the search | Examiner |
|---|---|---|
| The Hague | 10 November 2020 | Lommel, André |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons

& : member of the same patent family, corresponding document

EPO FORM 1503 03.82 (P04C01)

Europäisches
Patentamt

European
Patent Office

Office européen
des brevets

**EUROPEAN SEARCH REPORT**

Application Number

EP 20 17 8463

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | STEFANIE F. BUNK ET AL: "Does EEG activity during painful stimulation mirror more closely the noxious stimulus intensity or the subjective pain sensation?", SOMATOSENSORY AND MOTOR RESEARCH, vol. 35, no. 3-4, 2 October 2018 (2018-10-02), pages 192-198, XP55746614, US ISSN: 0899-0220, DOI: 10.1080/08990220.2018.1521790 * the whole document * | 1-17 | |
| X | PHILIPP TAESLER ET AL: "Prestimulus Theta Oscillations and Connectivity Modulate Pain Perception", THE JOURNAL OF NEUROSCIENCE, vol. 36, no. 18, 4 May 2016 (2016-05-04), pages 5026-5033, XP55746779, US ISSN: 0270-6474, DOI: 10.1523/JNEUROSCI.3325-15.2016 * the whole document * | 1-17 | TECHNICAL FIELDS SEARCHED (IPC) |

The present search report has been drawn up for all claims

| Place of search | Date of completion of the search | Examiner |
|---|---|---|
| The Hague | 10 November 2020 | Lommel, André |

CATEGORY OF CITED DOCUMENTS

X : particularly relevant if taken alone
Y : particularly relevant if combined with another
      document of the same category
A : technological background
O : non-written disclosure
P : intermediate document

T : theory or principle underlying the invention
E : earlier patent document, but published on, or
      after the filing date
D : document cited in the application
L : document cited for other reasons

& : member of the same patent family, corresponding
      document

EPO FORM 1503 03.82 (P04C01)

page 2 of 2

## ANNEX TO THE EUROPEAN SEARCH REPORT
## ON EUROPEAN PATENT APPLICATION NO.

EP 20 17 8463

This annex lists the patent family members relating to the patent documents cited in the above-mentioned European search report.
The members are as contained in the European Patent Office EDP file on
The European Patent Office is in no way liable for these particulars which are merely given for the purpose of information.

10-11-2020

| Patent document cited in search report | | Publication date | Patent family member(s) | | Publication date |
|---|---|---|---|---|---|
| US 2017311882 | A1 | 02-11-2017 | CA | 3062870 A1 | 02-11-2017 |
| | | | EP | 3490436 A1 | 05-06-2019 |
| | | | US | 2017311882 A1 | 02-11-2017 |
| | | | WO | 2017190044 A1 | 02-11-2017 |
| US 2014066739 | A1 | 06-03-2014 | NONE | | |
| US 2017135631 | A1 | 18-05-2017 | EP | 2432379 A1 | 28-03-2012 |
| | | | US | 8512240 B1 | 20-08-2013 |
| | | | US | 2013310660 A1 | 21-11-2013 |
| | | | US | 2017135631 A1 | 18-05-2017 |
| | | | WO | 2010134068 A1 | 25-11-2010 |
| US 2016278697 | A1 | 29-09-2016 | AU | 2003277305 A1 | 13-05-2004 |
| | | | EP | 1581146 A2 | 05-10-2005 |
| | | | US | 2004079372 A1 | 29-04-2004 |
| | | | US | 2006241562 A1 | 26-10-2006 |
| | | | US | 2010224188 A1 | 09-09-2010 |
| | | | US | 2011162645 A1 | 07-07-2011 |
| | | | US | 2013144183 A1 | 06-06-2013 |
| | | | US | 2016278697 A1 | 29-09-2016 |
| | | | WO | 2004037114 A2 | 06-05-2004 |
| US 2018228421 | A1 | 16-08-2018 | US | 2018228421 A1 | 16-08-2018 |
| | | | WO | 2017027703 A1 | 16-02-2017 |

EPO FORM P0459

For more details about this annex : see Official Journal of the European Patent Office, No. 12/82

**REFERENCES CITED IN THE DESCRIPTION**

**Non-patent literature cited in the description**

- **JONATHAN G. HARDMAN ; PHILIP M. HOPKINS ; MICHEL M. R. F. STRUYS.** the Oxford Textbook of Anaesthesia. Oxford University Press, 2017